(19) Europäisches Patentamt
European Patent Office
Office européen des brevets

(11) EP 2 278 009 A1

# (12) EUROPEAN PATENT APPLICATION

(43) Date of publication:
**26.01.2011 Bulletin 2011/04**

(21) Application number: **10179935.1**

(22) Date of filing: **27.03.2002**

(51) Int Cl.:
*C12N 15/31* (2006.01) *C12N 1/21* (2006.01)
*C12Q 1/68* (2006.01) *A61K 39/09* (2006.01)
*A61P 31/04* (2006.01) *C07K 14/315* (2006.01)
*C07K 16/12* (2006.01) *G01N 33/68* (2006.01)

(84) Designated Contracting States:
**AT BE CH CY DE DK ES FI FR GB GR IE IT LI LU MC NL PT SE TR**

(30) Priority: **27.03.2001 GB 0107658**

(62) Document number(s) of the earlier application(s) in accordance with Art. 76 EPC:
**05075713.7 / 1 630 230**
**02735782.1 / 1 373 513**

(71) Applicants:
- **Novartis Vaccines and Diagnostics S.r.l.**
  **53100 Siena (SI) (IT)**
- **J. Craig Venter Institute, Inc.**
  **Rockville, MD 20850 (US)**

(72) Inventors:
- **Masignani, Vega**
  **53100, Siena (IT)**
- **Tettelin, Herve**
  **Rockville, MD 20850 (US)**
- **Fraser, Claire**
  **Rockville, MD 20850 (US)**

(74) Representative: **Bullett, Rachel Margaret**
**Carpmaels & Ransford**
**One Southampton Row**
**London**
**WC1B 5HA (GB)**

Remarks:
This application was filed on 27-09-2010 as a divisional application to the application mentioned under INID code 62.

## (54) Streptococcus pneumoniae proteins and nucleic acids

(57) The invention provides proteins and nucleic acid sequences from *Streptococcus pneumoniae*. These are useful for the development of vaccines, diagnostics, and antibiotics.

EP 2 278 009 A1

SEQUENCE LISTING

<110> NOVARTIS VACCINES AND DIAGNOSTICS S.R.L.
<110> J. CRAIG VENTER INSTITUTE, INC.

<120> STREPTOCOCCUS PNEUMONIAE PROTEINS AND NUCLEIC ACIDS

<130> P055663EP

<150> EP05075713.7
<151> 2002-03-27

<150> GB-0107658.7
<151> 2001-03-27

<160> 2

<170> SeqWin99, version 1.03

<210> 1
<211> 1995
<212> DNA
<213> Streptococcus pneumoniae

<400> 1
atgaaatcaa tcaacaaatt tttaacaatg cttgctgcct tattactgac agcgagtagc 60
ctgttttcag ctgcaacagt ttttgcggct gggacgacaa caacatctgt taccgttcat 120
aaactattgg caacagatgg ggatatggat aaaattgcaa atgagttaga aacaggtaac 180
tatgctggta ataaagtggg tgttctacct gcaaatgcaa aagaaattgc cggtgttatg 240
ttcgtttgga caaatactaa taatgaaatt attgatgaaa atggccaaac tctaggagtg 300
aatattgatc cacaaacatt taaactctca ggggcaatgc cggcaactgc aatgaaaaaa 360
ttaacagaag ctgaaggagc taaatttaac acggcaaatt taccagctgc taagtataaa 420
atttatgaaa ttcacagttt atcaacttat gtcggtgaag atggagcaac cttaacaggt 480
tctaaagcag ttccaattga aattgaatta ccattgaacg atgttgtgga tgcgcatgtg 540
tatccaaaaa atacagaagc aaagccaaaa attgataaag atttcaaagg taaagcaaat 600
ccagatacac cacgtgtaga taaagataca cctgtgaacc accaagttgg agatgttgta 660
gagtacgaaa ttgttacaaa aattccagca cttgctaatt atgcaacagc aaactggagc 720
gatagaatga ctgaaggttt ggcattcaac aaaggtacag tgaaagtaac tgttgatgat 780
gttgcacttg aagcaggtga ttatgctcta acagaagtag caactggttt tgatttgaaa 840
ttaacagatg ctggtttagc taaagtgaat gaccaaaacg ctgaaaaaac tgtgaaaatc 900
acttattcgg caacattgaa tgacaaagca attgtagaag taccagaatc taatgatgta 960
acatttaact atggtaataa tccagatcac gggaatactc caaagccgaa taagccaaat 1020
gaaaacggcg atttgacatt gaccaagaca tgggttgatg ctacaggtgc accaattccg 1080
gctggagctg aagcaacgtt cgatttggtt aatgctcaga ctggtaaagt tgtacaaact 1140
gtaactttga caacagacaa aaatacagtt actgttaacg gattggataa aaatacagaa 1200
tataaattcg ttgaacgtag tataaaaggg tattcagcag attatcaaga aatcactaca 1260
gctggagaaa ttgctgtcaa gaactggaaa gacgaaaatc caaaaccact tgatccaaca 1320
gagccaaaag ttgttacata tggtaaaaag tttgtcaaag ttaatgataa agataatcgt 1380
ttagctgggg cagaatttgt aattgcaaat gctgataatg ctggtcaata tttagcacgt 1440
aaagcagata aagtgagtca agaagagaag cagttggttg ttacaacaaa ggatgcttta 1500
gatagagcag ttgctgctta taacgctctt actgcacaac aacaaactca gcaagaaaaa 1560
gagaaagttg acaaagctca agctgcttat aatgctgctg tgattgctgc caacaatgca 1620
tttgaatggg tggcagataa ggacaatgaa aatgttgtga aattagtttc tgatgcacaa 1680
ggtcgctttg aaattacagg ccttcttgca ggtacatatt acttagaaga aacaaaacag 1740
cctgctggtt atgcattact aactagccgt cagaaatttg aagtcactgc aacttcttat 1800
tcagcgactg gacaaggcat tgagtatact gctggttcag gtaaagatga cgctacaaaa 1860
gtagtcaaca aaaaaatcac tatcccacaa acgggtggta ttggtacaat tatctttgct 1920
gtagcggggg ctgcgattat gggtattgca gtgtacgcat atgttaaaaa caacaaagat 1980
gaggatcaac ttgct 1995

<210> 2
<211> 665
<212> PRT
<213> Streptococcus pneumoniae

<400> 2

```
Met Lys Ser Ile Asn Lys Phe Leu Thr Met Leu Ala Ala Leu Leu Leu
1               5                   10                  15

Thr Ala Ser Ser Leu Phe Ser Ala Ala Thr Val Phe Ala Ala Gly Thr
            20                  25                  30

Thr Thr Thr Ser Val Thr Val His Lys Leu Leu Ala Thr Asp Gly Asp
        35                  40                  45

Met Asp Lys Ile Ala Asn Glu Leu Glu Thr Gly Asn Tyr Ala Gly Asn
    50                  55                  60

Lys Val Gly Val Leu Pro Ala Asn Ala Lys Glu Ile Ala Gly Val Met
65                  70                  75                  80

Phe Val Trp Thr Asn Thr Asn Asn Glu Ile Ile Asp Glu Asn Gly Gln
                85                  90                  95

Thr Leu Gly Val Asn Ile Asp Pro Gln Thr Phe Lys Leu Ser Gly Ala
            100                 105                 110

Met Pro Ala Thr Ala Met Lys Lys Leu Thr Glu Ala Glu Gly Ala Lys
        115                 120                 125

Phe Asn Thr Ala Asn Leu Pro Ala Ala Lys Tyr Lys Ile Tyr Glu Ile
    130                 135                 140

His Ser Leu Ser Thr Tyr Val Gly Glu Asp Gly Ala Thr Leu Thr Gly
145                 150                 155                 160

Ser Lys Ala Val Pro Ile Glu Ile Glu Leu Pro Leu Asn Asp Val Val
                165                 170                 175

Asp Ala His Val Tyr Pro Lys Asn Thr Glu Ala Lys Pro Lys Ile Asp
            180                 185                 190

Lys Asp Phe Lys Gly Lys Ala Asn Pro Asp Thr Pro Arg Val Asp Lys
        195                 200                 205

Asp Thr Pro Val Asn His Gln Val Gly Asp Val Val Glu Tyr Glu Ile
    210                 215                 220

Val Thr Lys Ile Pro Ala Leu Ala Asn Tyr Ala Thr Ala Asn Trp Ser
225                 230                 235                 240

Asp Arg Met Thr Glu Gly Leu Ala Phe Asn Lys Gly Thr Val Lys Val
                245                 250                 255

Thr Val Asp Asp Val Ala Leu Glu Ala Gly Asp Tyr Ala Leu Thr Glu
            260                 265                 270

Val Ala Thr Gly Phe Asp Leu Lys Leu Thr Asp Ala Gly Leu Ala Lys
        275                 280                 285

Val Asn Asp Gln Asn Ala Glu Lys Thr Val Lys Ile Thr Tyr Ser Ala
```

```
        290                 295                 300

Thr Leu Asn Asp Lys Ala Ile Val Glu Val Pro Glu Ser Asn Asp Val
305                 310                 315                 320

Thr Phe Asn Tyr Gly Asn Asn Pro Asp His Gly Asn Thr Pro Lys Pro
                325                 330                 335

Asn Lys Pro Asn Glu Asn Gly Asp Leu Thr Leu Thr Lys Thr Trp Val
            340                 345                 350

Asp Ala Thr Gly Ala Pro Ile Pro Ala Gly Ala Glu Ala Thr Phe Asp
        355                 360                 365

Leu Val Asn Ala Gln Thr Gly Lys Val Val Gln Thr Val Thr Leu Thr
    370                 375                 380

Thr Asp Lys Asn Thr Val Thr Val Asn Gly Leu Asp Lys Asn Thr Glu
385                 390                 395                 400

Tyr Lys Phe Val Glu Arg Ser Ile Lys Gly Tyr Ser Ala Asp Tyr Gln
                405                 410                 415

Glu Ile Thr Thr Ala Gly Glu Ile Ala Val Lys Asn Trp Lys Asp Glu
            420                 425                 430

Asn Pro Lys Pro Leu Asp Pro Thr Glu Pro Lys Val Val Thr Tyr Gly
        435                 440                 445

Lys Lys Phe Val Lys Val Asn Asp Lys Asp Asn Arg Leu Ala Gly Ala
    450                 455                 460

Glu Phe Val Ile Ala Asn Ala Asp Asn Ala Gly Gln Tyr Leu Ala Arg
465                 470                 475                 480

Lys Ala Asp Lys Val Ser Gln Glu Glu Lys Gln Leu Val Val Thr Thr
                485                 490                 495

Lys Asp Ala Leu Asp Arg Ala Val Ala Ala Tyr Asn Ala Leu Thr Ala
            500                 505                 510

Gln Gln Gln Thr Gln Gln Glu Lys Glu Lys Val Asp Lys Ala Gln Ala
        515                 520                 525

Ala Tyr Asn Ala Ala Val Ile Ala Ala Asn Asn Ala Phe Glu Trp Val
    530                 535                 540

Ala Asp Lys Asp Asn Glu Asn Val Val Lys Leu Val Ser Asp Ala Gln
545                 550                 555                 560

Gly Arg Phe Glu Ile Thr Gly Leu Leu Ala Gly Thr Tyr Tyr Leu Glu
                565                 570                 575

Glu Thr Lys Gln Pro Ala Gly Tyr Ala Leu Leu Thr Ser Arg Gln Lys
            580                 585                 590

Phe Glu Val Thr Ala Thr Ser Tyr Ser Ala Thr Gly Gln Gly Ile Glu
        595                 600                 605

Tyr Thr Ala Gly Ser Gly Lys Asp Asp Ala Thr Lys Val Val Asn Lys
    610                 615                 620
```

```
Lys Ile Thr Ile Pro Gln Thr Gly Gly Ile Gly Thr Ile Ile Phe Ala
625                 630                 635                 640

Val Ala Gly Ala Ala Ile Met Gly Ile Ala Val Tyr Ala Tyr Val Lys
                645                 650                 655

Asn Asn Lys Asp Glu Asp Gln Leu Ala
            660                 665
```

## Description

### TECHNICAL FIELD

This invention relates to nucleic acid and proteins from the bacteria *Streptococcus pneumoniae.*

### BACKGROUND ART

*Streptococcus pneumoniae* is a Gram-positive spherical bacterium. It is the most common cause of acute bacterial meningitis in adults and in children over 5 years of age.

It is an object of the invention to provide materials for improving the prevention, detection and treatment of *Streptococcus pneumoniae* infections.

More specifically, it is an object of the invention to provide proteins which can be used in the development of vaccines. Further objects are to provide proteins and nucleic acid which can be used in the diagnosis of *S.pneumoniae* infection, to provide proteins and nucleic acid which can be used for the detection of *S.pneumoniae,* to provide nucleic acid which is useful for the expression of *S.pneumoniae* proteins, and to provide proteins which are useful targets for antibiotic research.

### DISCLOSURE OF THE INVENTION

The invention provides proteins comprising the *S.pneumoniae* amino acid sequence disclosed in the examples, SEQ ID NO:2. It also provides proteins comprising amino acid sequences having sequence identity to the *S.pneumoniae* amino acid sequence disclosed in the examples. Depending on the particular sequence, the degree of sequence identity is preferably greater than 50% (*e.g.* 60%, 70%, 80%, 90%, 95%, 99% or more). These proteins include homologs, orthologs, allelic variants and functional mutants. Typically, 50% identity or more between two proteins is considered to be an indication of functional equivalence. Identity between proteins is preferably determined by the Smith-Waterman homology search algorithm as implemented in the MPSRCH program (Oxford Molecular), using an affine gap search with parameters *gap open penalty=12* and *gap extension penalty=1.*

The invention further provides proteins comprising fragments of the *S.pneumoniae* amino acid sequence disclosed in the examples. The fragments should comprise at least *n* consecutive amino acids from the sequence and, depending on the particular sequence, *n* is 7 or more *(e.g.* 8, 10, 12, 14, 16, 18, 20, 30, 40, 50, 60, 70, 80, 90, 100 or more). Preferably the fragments comprise one or more epitopes from the sequence. Other preferred fragments are (a) the N-terminal signal peptides of the proteins disclosed in the examples, (b) the proteins disclosed in the examples, but without their N-terminal signal peptides, and (c) the proteins disclosed in the examples, but without their N-terminal amino acid residue.

The proteins of the invention can, of course, be prepared by various means (*e.g.* recombinant expression, purification from *S.pneumoniae,* chemical synthesis *etc.)* and in various forms *(e.g.* native, fusions, glycosylated, non-glycosylated *etc.).* They are preferably prepared in substantially pure form (*i.e.* substantially free from other streptococcal or host cell proteins). Proteins of the invention are preferably streptococcal proteins.

According to a further aspect, the invention provides antibodies which bind to these proteins. These may be polyclonal or monoclonal and may be produced by any suitable means. To increase compatibility with the human immune system, the antibodies may be chimeric or humanised (*e.g.* Breedveld (2000) Lancet 355(9205):735-740; Gorman & Clark (1990) Semin. Immunol. 2:457-466), or fully human antibodies may be used. The antibodies may include a detectable label *(e.g.* for diagnostic assays).

According to a further aspect, the invention provides nucleic acid comprising the *S.pneumoniae* nucleotide sequence disclosed in the examples. This nucleotide sequence is SEQ ID NO:1.

In addition, the invention provides nucleic acid comprising nucleotide sequences having sequence identity to the *S.pneumoniae* nucleotide sequence disclosed in the examples. Identity between sequences is preferably determined by the Smith-Waterman homology algorithm as described above.

Furthermore, the invention provides nucleic acid which can hybridise to the *S.pneumoniae* nucleic acid disclosed in the examples, preferably under "high stringency" conditions (*e.g.* 65°C in a 0.1xSSC, 0.5% SDS solution).

Nucleic acid comprising fragments of these sequences are also provided. These should comprise at least *n* consecutive nucleotides from the *S.pneumoniae* sequences and, depending on the particular sequence, *n* is 10 or more *(e.g.* 12, 14, 15, 18, 20, 25, 30, 35, 40, 50, 60, 70, 80, 90, 100, 150, 200 or more).

According to a further aspect, the invention provides nucleic acid encoding the proteins and protein fragments of the invention.

Nucleic acids of the invention can be used in hybridisation reactions (*e.g.* Northern or Southern blots, or in nucleic acid microarrays or 'gene chips') and amplification reactions (*e.g.* PCR, SDA, SSSR, LCR, NASBA, TMA) *etc.*

It should also be appreciated that the invention provides nucleic acid comprising sequences complementary to those described above (*e.g.* for antisense or probing purposes, or for use as primers).

Nucleic acid according to the invention can, of course, be prepared in many ways (*e.g.* by chemical synthesis, from genomic or cDNA libraries, from the organism itself *etc.)* and can take various forms (*e.g.* single stranded, double stranded, vectors, primers, probes *etc.).* The nucleic acid is preferably in substantially isolated form.

Nucleic acid according to the invention may be labelled *e.g.* with a radioactive or fluorescent label. This is particularly useful where it is to be used as a primer or probe *e.g.* in PCR, LCR, NASBA, TMA.

In addition, the term "nucleic acid" includes DNA and RNA, and also their analogues, such as those containing modified backbones, and also peptide nucleic acids (PNA) *etc.*

According to a further aspect, the invention provides vectors comprising nucleotide sequences of the invention (*e.g.* cloning or expression vectors) and host cells transformed with such vectors.

According to a further aspect, the invention provides compositions comprising protein, antibody, and/or nucleic acid according to the invention. These compositions may be suitable as immunogenic compositions, for instance, or as diagnostic reagents, or as vaccines.

The invention also provides nucleic acid, protein, or antibody according to the invention for use as medicaments (*e.g.* as immunogenic compositions or vaccines) or as diagnostic reagents. It also provides the use of nucleic acid, protein, or antibody according to the invention in the manufacture of: (i) a medicament for treating or preventing infection due to streptococcus; (ii) a diagnostic reagent for detecting the presence of streptococcus or of antibodies raised against streptococcus; and/or (iii) a reagent which can raise antibodies against streptococcus. Said streptococcus may be any species, group or strain, but is preferably *S.pneumoniae,* particularly a type 4 strain. The disease may be meningitis, pneumonia, sepsis, otitis media or an ear infection.

The invention also provides a method of treating a patient, comprising administering to the patient a therapeutically effective amount of nucleic acid, protein, and/or antibody of the invention. The patient may either be at risk from the disease themselves or may be a pregnant woman ('maternal immunisation' *e.g.* Glezen & Alpers (1999) Clin. Infect. Dis. 28:219-224).

Administration of protein antigens is a preferred method of treatment for inducing immunity.

Administration of antibodies of the invention is another preferred method of treatment. This method of passive immunisation is particularly useful for newborn children or for pregnant women. This method will typically use monoclonal antibodies, which will be humanised or fully human.

The invention also provides a kit comprising primers (*e.g.* PCR primers) for amplifying a target sequence contained within a *Streptococcus* (*e.g. S.pneumoniae*) nucleic acid sequence, the kit comprising a first primer and a second primer, wherein the first primer is substantially complementary to said target sequence and the second primer is substantially complementary to a complement of said target sequence, wherein the parts of said primers which have substantial complementarity define the termini of the target sequence to be amplified. The first primer and/or the second primer may include a detectable label (*e.g.* a fluorescent label).

The invention also provides a kit comprising first and second single-stranded oligonucleotides which allow amplification of a *Streptococcus* (*e.g. S.pneumoniae*) template nucleic acid sequence contained in a single- or double-stranded nucleic acid (or mixture thereof), wherein: (a) the first oligonucleotide comprises a primer sequence which is substantially complementary to said template nucleic acid sequence; (b) the second oligonucleotide comprises a primer sequence which is substantially complementary to the complement of said template nucleic acid sequence; (c) the first oligonucleotide and/or the second oligonucleotide comprise(s) sequence which is not complementary to said template nucleic acid; and (d) said primer sequences define the termini of the template sequence to be amplified. The non-complementary sequence(s) of feature (c) are preferably upstream of (i. e. 5' to) the primer sequences. One or both of these (c) sequences may comprise a restriction site (*e.g.* EP-B-0509612) or a promoter sequence (*e.g.* EP-B-0505012). The first oligonucleotide and/or the second oligonucleotide may include a detectable label (*e.g.* a fluorescent label).

The template sequence may be any part of a genome sequence. For example, it could be a rRNA gene or a protein-coding gene. The template sequence is preferably specific to *S.pneumoniae.*

The invention also provides a computer-readable medium (*e.g.* a floppy disk, a hard disk, a CD-ROM, a DVD *etc.)* and/or a computer database containing one or more of the sequences in the sequence listing.

The invention also provides a hybrid protein represented by the formula $NH_2$-A-[-X-L-]$_n$-B-COOH, wherein X is an amino acid sequence of the invention as described above, L is an optional linker amino acid sequence, A is an optional N-terminal amino acid sequence, B is an optional C-terminal amino acid sequence, and *n* is an integer greater than 1. The value of *n* is between 2 and x, and the value of *x* is typically 3, 4, 5, 6, 7, 8, 9 or 10. Preferably *n* is 2, 3 or 4; it is more preferably 2 or 3; most preferably, *n* = 2. For each *n* instances, -X- may be the same or different. For each *n* instances of [-X-L-], linker amino acid sequence -L- may be present or absent. For instance, when *n*=2 the hybrid may be $NH_2$-$X_1$-$L_1$-$X_2$-$L_2$-COOH, $NH_2$-$X_1$-$X_2$-COOH, $NH_2$-$X_1$-$L_1$-$X_2$-COOH, $NH_2$-$X_1$-$X_2$-$L_2$-COOH, *etc.* Linker amino acid sequence(s) -L- will typically be short *(e.g.* 20 or fewer amino acids *i.e.* 19, 18, 17, 16, 15, 14, 13, 12, 11, 10, 9, 8, 7, 6, 5, 4, 3, 2, 1). Examples include short peptide sequences which facilitate cloning, poly-glycine linkers (*i.e.* $Gly_n$ where *n*

= 2, 3, 4, 5, 6, 7, 8, 9, 10 or more), and histidine tags (*i.e.* $His_n$ where $n$ = 3, 4, 5, 6, 7, 8, 9, 10 or more). Other suitable linker amino acid sequences will be apparent to those skilled in the art. -A- and -B- are optional sequences which will typically be short (*e.g.* 40 or fewer amino acids *i.e.* 39, 38, 37, 36, 35, 34, 33, 32, 31, 30, 29, 28, 27, 26, 25, 24, 23, 22, 21, 20, 19, 18, 17, 16, 15, 14, 13, 12, 11, 10, 9, 8, 7, 6, 5, 4, 3, 2, 1). Examples include leader sequences to direct protein trafficking, or short peptide sequences which facilitate cloning or purification (*e.g.* histidine tags *i.e.* $His_n$ where $n$ = 3, 4, 5, 6, 7, 8, 9, 10 or more). Other suitable N-terminal and C-terminal amino acid sequences will be apparent to those skilled in the art. In some embodiments, each X will be a *S.pneumoniae* sequence; in others, mixtures of *S.pneumoniae, S.pyogenes* and/or *S.agalactiae* sequences will be used [see even SEQ IDs 2 to 10966 of PCT/GB01/04789 for suitable sequences].

In some embodiments of the invention, the proteins and nucleic acids of the invention share sequence identity with the 2043 ORF sequences from the avirulent R6 strain of *S.pneumoniae* disclosed by Hoskins et al. [J Bacteriol (2001) 183:5709-17]. In other embodiments, the invention does not encompass sequences consisting of one of the 2043 ORFs specifically disclosed by Hoskins *et al.*

According to further aspects, the invention provides various processes.

A process for producing proteins of the invention is provided, comprising the step of culturing a host cell of to the invention under conditions which induce protein expression.

A process for producing protein or nucleic acid of the invention is provided, wherein the protein or nucleic acid is synthesised in part or in whole using chemical means.

A process for detecting polynucleotides of the invention is provided, comprising the steps of: (a) contacting a nucleic probe according to the invention with a biological sample under hybridising conditions to form duplexes; and (b) detecting said duplexes.

A process for detecting *Streptococcus* in a biological sample is also provided, comprising the step of contacting nucleic according to the invention with the biological sample under hybridising conditions. The process may involve nucleic acid amplification (*e.g.* PCR, SDA, SSSR, LCR, NASBA, TMA *etc.)* or hybridisation (*e.g.* microarrays, blots, hybridisation with a probe in solution *etc.*). PCR detection of *S.pneumoniae* in clinical samples has previously been reported [see *e.g.* Cherian et al. (1998) J. Clin. Microbiol. 36:3605-3608; Kearns et al. (1999) J. Clin. Microbiol. 37:3434; Matsumura, abstract D-25, 38th Annual ICAAC].

A process for detecting proteins of the invention is provided, comprising the steps of: (a) contacting an antibody of the invention with a biological sample under conditions suitable for the formation of an antibody-antigen complexes; and (b) detecting said complexes.

A process for identifying an amino acid sequence is provided, comprising the step of searching for putative open reading frames or protein-coding regions within a genome sequence of *S.pneumoniae.* This will typically involve *in silico* searching the sequence for an initiation codon and for an in-frame termination codon in the downstream sequence. The region between these initiation and termination codons is a putative protein-coding sequence. Typically, all six possible reading frames will be searched. Suitable software for such analysis includes ORFFINDER (NCBI), GENEMARK [Borodovsky & McIninch (1993) Computers Chem. 17:122-133), GLIMMER [Salzberg et al. (1998) Nucleic Acids Res. 26:544-548; Salzberg et al. (1999) Genomics 59:24-31; Delcher et al. (1999) Nucleic Acids Res. 27:4636-4641], or other software which uses Markov models [*e.g.* Shmatkov et al. (1999) Bioinformatics 15:874-876]. The invention also provides a protein comprising the identified amino acid sequence. These proteins can then expressed using conventional techniques.

The invention also provides a process for determining whether a test compound binds to a protein of the invention. If a test compound binds to a protein of the invention and this binding inhibits the life cycle of the *S.pneumoniae* bacterium, then the test compound can be used as an antibiotic or as a lead compound for the design of antibiotics. The process will typically comprise the steps of contacting a test compound with a protein of the invention, and determining whether the test compound binds to said protein. Preferred proteins of the invention for use in these processes are enzymes (*e.g.* tRNA synthetases), membrane transporters and ribosomal proteins. Suitable test compounds include proteins, polypeptides, carbohydrates, lipids, nucleic acids (*e.g.* DNA, RNA, and modified forms thereof), as well as small organic compounds (*e.g.* MW between 200 and 2000 Da). The test compounds may be provided individually, but will typically be part of a library (*e.g.* a combinatorial library). Methods for detecting a binding interaction include NMR, filter-binding assays, gel-retardation assays, displacement assays, surface plasmon resonance, reverse two-hybrid *etc.* A compound which binds to a protein of the invention can be tested for antibiotic activity by contacting the compound with GBS bacteria and then monitoring for inhibition of growth. The invention also provides a compound identified using these methods.

The invention also provides a composition comprising a protein or the invention and one or more of the following antigens:

- a protein antigen from *Helicobacter pylori* such as VacA, CagA, NAP, HopX, HopY *[e.g.* WO98/04702] and/or urease.

- a protein antigen from *N.meningitidis* serogroup B, such as those in WO99/24578, WO99/36544, WO99/57280, WO00/22430, Tettelin et al. (2000) Science 287:1809-1815, Pizza et al. (2000) Science 287:1816-1820 and WO96/29412, with protein '287' and derivatives being particularly preferred.

- an outer-membrane vesicle (OMV) preparation from *N.meningitidis* serogroup B, such as those disclosed in WO01/52885; Bjune et al. (1991) Lancet 338(8775):1093-1096; Fukasawa et al. (1999) Vaccine 17:2951-2958; Rosenqvist et al. (1998) Dev. Biol. Stand. 92:323-333 *etc.*

- a saccharide antigen from *N.meningitidis* serogroup A, C, W135 and/or Y, such as the oligosaccharide disclosed in Costantino et al. (1992) Vaccine 10:691-698from serogroup C [see also Costantino et al. (1999) Vaccine 17: 1251-1263].

- a saccharide antigen from *Streptococcus pneumoniae* [*e.g.* Watson (2000) Pediatr Infect Dis J 19:331-332; Rubin (2000) Pediatr Clin North Am 47:269-285, v; Jedrzejas (2001) Microbiol Mol Biol Rev 65:187-207].

- an antigen from hepatitis A virus, such as inactivated virus [*e.g.* Bell (2000) Pediatr Infect Dis J 19:1187-1188; Iwarson (1995) APMIS 103:321-326].

- an antigen from hepatitis B virus, such as the surface and/or core antigens [*e.g.* Gerlich et al. (1990) Vaccine 8 Suppl:S63-68 & 79-80].

- an antigen from hepatitis C virus [*e.g.* Hsu et al. (1999) Clin Liver Dis 3:901-915].

- an antigen from *Bordetella pertussis,* such as pertussis holotoxin (PT) and filamentous haemagglutinin (FHA) from *B.pertussis,* optionally also in combination with pertactin and/or agglutinogens 2 and 3 [*e.g.* Gustafsson et al. (1996) N. Engl. J. Med. 334:349-355; Rappuoli et al. (1991) TIBTECH 9:232-238].

- a diphtheria antigen, such as a diphtheria toxoid [*e.g.* chapter 3 of Vaccines (1988) eds. Plotkin & Mortimer. ISBN 0-7216-1946-0] *e.g.* the $CRM_{197}$ mutant *[e.g.* Del Guidice et al. (1998) Molecular Aspects of Medicine 19:1-70].

- a tetanus antigen, such as a tetanus toxoid [*e.g.* chapter 4 of Plotkin & Mortimer].

- a saccharide antigen from *Haemophilus influenzae* B.

- an antigen from *N.gonorrhoeae* [*e.g.* WO99/24578, WO99/36544, WO99/57280].

- an antigen from *Chlamydia pneumoniae* [*e.g.* WO02/02606; Kalman et al. (1999) Nature Genetics 21:385-389; Read et al. (2000) Nucleic Acids Res 28:1397-406; Shirai et al. (2000) J. Infect. Dis. 181(Suppl 3):S524-S527; WO99/27105; WO00/27994; WO00/37494].

- an antigen from *S.agalactiae* [*e.g.* PCT/GB01/04789]

- an antigen from *S.pyogenes* [*e.g.* PCT/GB01/04789]

- an antigen from *Chlamydia trachomatis* [*e.g.* WO99/28475].

- an antigen from *Porphyromonas gingivalis* [*e.g.* Ross et al. (2001) Vaccine 19:4135-4142].

- polio antigen(s) [*e.g.* Sutter et al. (2000) Pediatr Clin North Am 47:287-308; Zimmerman & Spann (1999) Am Fam Physician 59:113-118, 125-126] such as IPV or OPV.

- rabies antigen(s) *[e.g.* Dreesen (1997) Vaccine 15 Suppl:S2-6] such as lyophilised inactivated virus [*e.g.* MMWR Morb Mortal Wkly Rep 1998 Jan 16;47(1):12, 19; RabAvert™].

- measles, mumps and/or rubella antigens [*e.g.* chapters 9, 10 & 11 of Plotkin & Mortimer].

- influenza antigen(s) [*e.g.* chapter 19 of Plotkin & Mortimer], such as the haemagglutinin and/or neuraminidase surface proteins.

- an antigen from *Moraxella catarrhalis* [*e.g.* McMichael (2000) Vaccine 19 Suppl 1:S101-107].

- an antigen from *Staphylococcus aureus* [*e.g.* Kuroda et al. (2001) Lancet 357(9264):1225-1240; see also pages 1218-1219].

Where a saccharide or carbohydrate antigen is included, it is preferably conjugated to a carrier protein in order to enhance immunogenicity [*e.g.* Ramsay et al. (2001) Lancet 357(9251):195-196; Lindberg (1999) Vaccine 17 Suppl 2:S28-36; Conjugate Vaccines (eds. Cruse et al.) ISBN 3805549326, particularly vol. 10:48-114 *etc.*]. Preferred carrier proteins are bacterial toxins or toxoids, such as diphtheria or tetanus toxoids. The $CRM_{197}$ diphtheria toxoid is particularly preferred. Other suitable carrier proteins include the *N.meningitidis* outer membrane protein [*e.g.* EP-0372501], synthetic peptides [*e.g.* EP-0378881, EP-0427347], heat shock proteins [*e.g.* WO93/17712], pertussis proteins [*e.g.* WO98/58668; EP-0471177], protein D from *H.influenzae* [*e.g.* WO00/56360], toxin A or B from *C.difficile* [*e.g.* WO00/61761], *etc.* Any suitable conjugation reaction can be used, with any suitable linker where necessary.

Toxic protein antigens may be detoxified where necessary (*e.g.* detoxification of pertussis toxin by chemical and/or genetic means).

Where a diphtheria antigen is included in the composition it is preferred also to include tetanus antigen and pertussis antigens. Similarly, where a tetanus antigen is included it is preferred also to include diphtheria and pertussis antigens. Similarly, where a pertussis antigen is included it is preferred also to include diphtheria and tetanus antigens.

Antigens are preferably adsorbed to an aluminium salt.

Antigens in the composition will typically be present at a concentration of at least 1μg/ml each. In general, the concentration of any given antigen will be sufficient to elicit an immune response against that antigen.

The invention also provides compositions comprising two or more *(e.g.* 3, 4, 5) proteins of the invention.

A summary of standard techniques and procedures which may be employed to perform the invention (*e.g.* to utilise the disclosed sequences for vaccination or diagnostic purposes) follows. This summary is not a limitation on the invention but, rather, gives examples that may be used, but are not required.

*General*

The practice of the present invention will employ, unless otherwise indicated, conventional techniques of molecular biology, microbiology, recombinant DNA, and immunology, which are within the skill of the art. Such techniques are explained fully in the literature *eg.* Sambrook Molecular Cloning; A Laboratory Manual, Second Edition (1989); DNA Cloning, Volumes I and II (D.N Glover ed. 1985); Oligonucleotide Synthesis (M.J. Gait ed, 1984); Nucleic Acid Hybridization (B.D. Hames & S.J. Higgins eds. 1984); Transcription and Translation (B.D. Hames & S.J. Higgins eds. 1984); Animal Cell Culture (R.I. Freshney ed. 1986); Immobilized Cells and Enzymes (IRL Press, 1986); B. Perbal, A Practical Guide to Molecular Cloning (1984); the Methods in Enzymology series (Academic Press, Inc.), especially volumes 154 & 155; Gene Transfer Vectors for Mammalian Cells (J.H. Miller and M.P. Calos eds. 1987, Cold Spring Harbor Laboratory); Mayer and Walker, eds. (1987), Immunochemical Methods in Cell and Molecular Biology (Academic Press, London); Scopes, (1987) Protein Purification: Principles and Practice, Second Edition (Springer-Verlag, N.Y.), and Handbook of Experimental Immunology, Volumes I-IV (D.M. Weir and C. C. Blackwell eds 1986).

Standard abbreviations for nucleotides and amino acids are used in this specification.

*Definitions*

A composition containing X is "substantially free of" Y when at least 85% by weight of the total X+Y in the composition is X. Preferably, X comprises at least about 90% by weight of thetotal of X+Y in the composition, more preferably at least about 95% or even 99% by weight.

The term "comprising" means "including" as well as "consisting" *e.g.* a composition "comprising" X may consist exclusively of X or may include something additional *e.g.* X + Y.

The term "heterologous" refers to two biological components that are not found together in nature. The components may be host cells, genes, or regulatory regions, such as promoters. Although the heterologous components are not found together in nature, they can function together, as when a promoter heterologous to a gene is operably linked to the gene. Another example is where a streptococcus sequence is heterologous to a mouse host cell. A further examples would be two epitopes from the same or different proteins which have been assembled in a single protein in an arrangement not found in nature

An "origin of replication" is a polynucleotide sequence that initiates and regulates replication of polynucleotides, such as an expression vector. The origin of replication behaves as an autonomous unit of polynucleotide replication within a cell, capable of replication under its own control. An origin of replication may be needed for a vector to replicate in a particular host cell. With certain origins of replication, an expression vector can be reproduced at a high copy number in the presence of the appropriate proteins within the cell. Examples of origins are the autonomously replicating sequences, which are effective in yeast; and the viral T-antigen, effective in COS-7 cells.

A "mutant" sequence is defined as DNA, RNA or amino acid sequence differing from but having sequence identity with the native or disclosed sequence. Depending on the particular sequence, the degree of sequence identity between the native or disclosed sequence and the mutant sequence is preferably greater than 50% (*eg*. 60%, 70%, 80%, 90%, 95%, 99% or more, calculated using the Smith-Waterman algorithm as described above). As used herein, an "allelic variant" of a nucleic acid molecule, or region, for which nucleic acid sequence is provided herein is a nucleic acid molecule, or region, that occurs essentially at the same locus in the genome of another or second isolate, and that, due to natural variation caused by, for example, mutation or recombination, has a similar but not identical nucleic acid sequence. A coding region allelic variant typically encodes a protein having similar activity to that of the protein encoded by the gene to which it is being compared. An allelic variant can also comprise an alteration in the 5' or 3' untranslated regions of the gene, such as in regulatory control regions (*eg*. see US patent 5,753,235).

*Expression systems*

The streptococcus nucleotide sequences can be expressed in a variety of different expression systems; for example those used with mammalian cells, baculoviruses, plants, bacteria, and yeast.

i. Mammalian Systems

Mammalian expression systems are known in the art. A mammalian promoter is any DNA sequence capable of binding mammalian RNA polymerase and initiating the downstream (3') transcription of a coding sequence (*eg*. structural gene) into mRNA. A promoter will have a transcription initiating region, which is usually placed proximal to the 5' end of the coding sequence, and a TATA box, usuallylocated 25-30 base pairs (bp) upstream of the transcription initiation site. The TATA box is thought to direct RNA polymerase II to begin RNA synthesis at the correct site. A mammalian promoter will also contain an upstream promoter element, usually located within 100 to 200 bp upstream of the TATA box. An upstream promoter element determines the rate at which transcription is initiated and can act in either orientation [Sambrook et al. (1989) "Expression of Cloned Genes in Mammalian Cells." In Molecular Cloning: A Laboratory Manual, 2nd ed.*].*

Mammalian viral genes are often highly expressed and have a broad host range; therefore sequences encoding mammalian viral genes provide particularly useful promoter sequences. Examples include the SV40 early promoter, mouse mammary tumor virus LTR promoter, adenovirus major late promoter (Ad MLP), and herpes simplex virus promoter. In addition, sequences derived from non-viral genes, such as the murine metallotheionein gene, also provide useful promoter sequences. Expression may be either constitutive or regulated (inducible), depending on the promoter can be induced with glucocorticoid in hormoneresponsive cells.

The presence of an enhancer element (enhancer), combined with the promoter elements described above, will usually increase expression levels. An enhancer is a regulatory DNA sequence that can stimulate transcription up to 1000-fold when linked to homologous or heterologous promoters, with synthesis beginning at the normal RNA start site. Enhancers are also active when they are placed upstream or downstream from the transcription initiation site, in either normal or flipped orientation, or at a distance of more than 1000 nucleotides from the promoter [Maniatis et al. (1987) Science 236:1237; Alberts et al. (1989) Molecular Biology of the Cell, 2nd ed.]. Enhancer elements derived from viruses may be particularly useful, because they usually have a broader host range. Examples include the SV40 early gene enhancer [Dijkema et al (1985) EMBO J. 4:761] and the enhancer/promoters derived from the long terminal repeat (LTR) of the Rous Sarcoma Virus [Gorman et al. (1982b) Proc. Natl. Acad. Sci. 79:6777] and from human cytomegalovirus [Boshart et al. (1985) Cell 41:521]. Additionally, some enhancers are regulatable and become active only in the presence of an inducer, such as a hormone or metal ion [Sassone-Corsi and Borelli (1986) Trends Genet. 2:215; Maniatis et al. (1987) Science 236:1237].

A DNA molecule may be expressed intracellularly in mammalian cells. A promoter sequence may be directly linked with the DNA molecule, in which case the first amino acid at the N-terminus of the recombinant protein will always be a methionine, which is encoded by the ATG start codon. If desired, the N-terminus may be cleaved from the protein by *in vitro* incubation with cyanogen bromide.

Alternatively, foreign proteins can also be secreted from the cell into the growth media by creating chimeric DNA molecules that encode a fusion protein comprised of a leader sequence fragment that provides for secretion of the foreign protein in mammalian cells. Preferably, there are processing sites encoded between the leader fragment and the foreign gene that can be cleaved either *in vivo* or *in vitro.* The leader sequence fragment usually encodes a signal peptide comprised of hydrophobic amino acids which direct the secretion of the protein from the cell. The adenovirus triparite leader is an example of a leader sequence that provides for secretion of a foreign protein in mammalian cells.

Usually, transcription termination and polyadenylation sequences recognized by mammalian cells are regulatory regions located 3' to the translation stop codon and thus, together with the promoter elements, flank the coding sequence. The 3' terminus of the mature mRNA is formed by site-specific post-transcriptional cleavage and polyadenylation [Birnstiel et al. (1985) Cell 41:349; Proudfoot and Whitelaw (1988) "Termination and 3' end processing of eukaryotic RNA. In Transcription and splicing (ed. B.D. Hames and D.M. Glover); Proudfoot (1989) Trends Biochem. Sci. 14:105]. These sequences direct the transcription of an mRNA which can be translated into the polypeptide encoded by the DNA. Examples of transcription terminater/polyadenylation signals include those derived from SV40 [Sambrook et al (1989) "Expression of cloned genes in cultured mammalian cells." In Molecular Cloning: A Laboratory Manual].

Usually, the above described components, comprising a promoter, polyadenylation signal, and transcription termination sequence are put together into expression constructs. Enhancers, introns with functional splice donor and acceptor sites, and leader sequences may also be included in an expression construct, if desired. Expression constructs are often maintained in a replicon, such as an extrachromosomal element (*eg.* plasmids) capable of stable maintenance in a host, such as mammalian cells or bacteria. Mammalian replication systems include those derived from animal viruses, which require trans-acting factors to replicate. For example, plasmids containing the replication systems of papovaviruses, such as SV40 [Gluzman (1981) Cell 23:175] or polyomavirus, replicate to extremely high copy number in the presence of the appropriate viral T antigen. Additional examples of mammalian replicons include those derived from bovine papillomavirus and Epstein-Barr virus. Additionally, the replicon may have two replicaton systems, thus allowing it to be maintained, for example, in mammalian cells for expression and in a prokaryotic host for cloning and amplification. Examples of such mammalian-bacteria shuttle vectors include pMT2 [Kaufman et al. (1989) Mol. Cell. Biol. 9:946] and pHEBO [Shimizu et al. (1986) Mol. Cell. Biol. 6:1074].

The transformation procedure used depends upon the host to be transformed. Methods for introduction of heterologous polynucleotides into mammalian cells are known in the art and include dextran-mediated transfection, calcium phosphate precipitation, polybrene mediated transfection, protoplast fusion, electroporation, encapsulation of the polynucleotide(s) in liposomes, and direct microinjection of the DNA into nuclei.

Mammalian cell lines available as hosts for expression are known in the art and include many immortalized cell lines available from the American Type Culture Collection (ATCC), including but not limited to, Chinese hamster ovary (CHO) cells, HeLa cells, baby hamster kidney (BHK) cells, monkey kidney cells (COS), human hepatocellular carcinoma cells (*eg.* Hep G2), and a number of other cell lines.

ii. Baculovirus Systems

The polynucleotide encoding the protein can also be inserted into a suitable insect expression vector, and is operably linked to the control elements within that vector. Vector construction employs techniques which are known in the art. Generally, the components of the expression system include a transfer vector, usually a bacterial plasmid, which contains both a fragment of the baculovirus genome, and a convenient restriction site for insertion of the heterologous gene or genes to be expressed; a wild type baculovirus with a sequence homologous to the baculovirus-specific fragment in the transfer vector (this allows for the homologous recombination of the heterologous gene in to the baculovirus genome); and appropriate insect host cells and growth media.

After inserting the DNA sequence encoding the protein into the transfer vector, the vector and the wild type viral genome are transfected into an insect host cell where the vector and viral genome are allowed to recombine. The packaged recombinant virus is expressed and recombinant plaques are identified and purified. Materials and methods for baculovirus/insect cell expression systems are commercially available in kit form from, *inter alia,* Invitrogen, San Diego CA ("MaxBac" kit). These techniques are generally known to those skilled in the art and fully described in Summers and Smith, Texas Agricultural Experiment Station Bulletin No. 1555 (1987) (hereinafter "Summers and Smith").

Prior to inserting the DNA sequence encoding the protein into the baculovirus genome, the above described components, comprising a promoter, leader (if desired), coding sequence, and transcription termination sequence, are usually assembled into an intermediate transplacement construct (transfer vector). This may contain a single gene and operably linked regulatory elements; multiple genes, each with its owned set of operably linked regulatory elements; or multiple genes, regulated by the same set of regulatory elements. Intermediate transplacement constructs are often maintained in a replicon, such as an extra-chromosomal element (*e.g.* plasmids) capable of stable maintenance in a host, such as a bacterium. The replicon will have a replication system, thus allowing it to be maintained in a suitable host for cloning and amplification.

Currently, the most commonly used transfer vector for introducing foreign genes into AcNPV is pAc373. Many other vectors, known to those of skill in the art, have also been designed. These include, for example, pVL985 (which alters the polyhedrin start codon from ATG to ATT, and which introduces a BamHI cloning site 32 basepairs downstream from the ATT; see Luckow and Summers, *Virology* (1989) *17*:31.

The plasmid usually also contains the polyhedrin polyadenylation signal (Miller et al. (1988) Ann. Rev. Microbiol., 42:177) and a prokaryotic ampicillin-resistance (*amp*) gene and origin of replication for selection and propagation in *E. coli.*

Baculovirus transfer vectors usually contain a baculovirus promoter. A baculovirus promoter is any DNA sequence capable of binding a baculovirus RNA polymerase and initiating the downstream (5' to 3') transcription of a coding sequence (*eg*. structural gene) into mRNA. A promoter will have a transcription initiation region which is usually placed proximal to the 5' end of the coding sequence. This transcription initiation region usually includes an RNA polymerase binding site and a transcription initiation site. A baculovirus transfer vector may also have a second domain called an enhancer, which, if present, is usually distal to the structural gene. Expression may be either regulated or constitutive.

Structural genes, abundantly transcribed at late times in a viral infection cycle, provide particularly useful promoter sequences. Examples include sequences derived from the gene encoding the viral polyhedron protein, Friesen et al., (1986) "The Regulation of Baculovirus Gene Expression," in: The Molecular Biology of Baculoviruses (ed. Walter Doerfler); EPO Publ. Nos. 127 839 and 155 476; and the gene encoding the p10 protein, Vlak et al., (1988), J. Gen. Virol. 69:765.

DNA encoding suitable signal sequences can be derived from genes for secreted insect or baculovirus proteins, such as the baculovirus polyhedrin gene (Carbonell et al. (1988) Gene, 73:409). Alternatively, since the signals for mammalian cell posttranslational modifications (such as signal peptide cleavage, proteolytic cleavage, and phosphorylation) appear to be recognized by insect cells, and the signals required for secretion and nuclear accumulation also appear to be conserved between the invertebrate cells and vertebrate cells, leaders of non-insect origin, such as those derived from genes encoding human $\alpha$-interferon, Maeda et al., (1985), Nature 315:592; human gastrin-releasing peptide, Lebacq-Verheyden et al., (1988), Molec. Cell. Biol. 8:3129; human IL-2, Smith et al., (1985) Proc. Nat'l Acad. Sci. USA, 82:8404; mouse IL-3, (Miyajima et al., (1987) Gene 58:273; and human glucocerebrosidase, Martin et al. (1988) DNA, 7:99, can also be used to provide for secretion in insects.

A recombinant polypeptide or polyprotein may be expressed intracellularly or, if it is expressed with the proper regulatory sequences, it can be secreted. Good intracellular expression of nonfused foreign proteins usually requires heterologous genes that ideally have a short leader sequence containing suitable translation initiation signals preceding an ATG start signal. If desired, methionine at the Nterminus may be cleaved from the mature protein by *in vitro* incubation with cyanogen bromide.

Alternatively, recombinant polyproteins or proteins which are not naturally secreted can be secreted from the insect cell by creating chimeric DNA molecules that encode a fusion protein comprised of a leader sequence fragment that provides for secretion of the foreign protein in insects. The leader sequence fragment usually encodes a signal peptide comprised of hydrophobic amino acids which direct the translocation of the protein into the endoplasmic reticulum.

After insertion of the DNA sequence and/or the gene encoding the expression product precursor of the protein, an insect cell host is co-transformed with the heterologous DNA of the transfer vector and the genomic DNA of wild type baculovirus -- usually by co-transfection. The promoter and transcription termination sequence of the construct will usually comprise a 2-5kb section of the baculovirus genome. Methods for introducing heterologous DNA into the desired site in the baculovirus virus are known in the art. (See Summers and Smith *supra*; Ju et al. (1987); Smith et al., Mol. Cell. Biol. (1983) 3:2156; and Luckow and Summers (1989)). For example, the insertion can be into a gene such as the polyhedrin gene, by homologous double crossover recombination; insertion can also be into a restriction enzyme site engineered into the desired baculovirus gene. Miller et al., (1989), Bioessays 4:91.The DNA sequence, when cloned in place of the polyhedrin gene in the expression vector, is flanked both 5' and 3' by polyhedrin-specific sequences and is positioned downstream of the polyhedrin promoter.

The newly formed baculovirus expression vector is subsequently packaged into an infectious recombinant baculovirus. Homologous recombination occurs at low frequency (between about 1% and about 5%);thus, the majority of the virus produced after cotransfection is still wild-type virus. Therefore, a method is necessary to identify recombinant viruses. An advantage of the expression system is a visual screen allowing recombinant viruses to be distinguished. The polyhedrin protein, which is produced by the native virus, is produced at very high levels in the nuclei of infected cells at late times after viral infection. Accumulated polyhedrin protein forms occlusion bodies that also contain embedded particles. These occlusion bodies, up to 15 $\mu$m in size, are highly refractile, giving them a bright shiny appearance that is readily visualized under the light microscope. Cells infected with recombinant viruses lack occlusion bodies. To distinguish recombinantvirus from wild-type virus, the transfection supernatant is plaqued onto a monolayer of insect cells by techniques known to those skilled in the art. Namely, the plaques are screened under the light microscope for the presence (indicative of wild-type virus) or absence (indicative of recombinant virus) of occlusion bodies. "Current Protocols in Microbiology" Vol. 2 (Ausubel et al. eds) at 16.8 (Supp. 10, 1990); Summers and Smith, *supra*; Miller et al. (1989).

Recombinant baculovirus expression vectors have been developed for infection into several insect cells. For example, recombinant baculoviruses have been developed for, *inter alia*: *Aedes aegypti , Autographa californica, Bombyx mori, Drosophila melanogaster, Spodoptera frugiperda,* and *Trichoplusia ni* (WO 89/046699; Carbonell et al., (1985) J. Virol. 56:153; Wright (1986) Nature 321:718; Smith et al., (1983) Mol. Cell. Biol. 3:2156; and see generally, Fraser, et al. (1989) In Vitro Cell. Dev. Biol. 25:225).

Cells and cell culture media are commercially available for both direct and fusion expression of heterologous polypeptides in a baculovirus/expression system; cell culture technology is generally known to those skilled in the art.

*See, eg.* Summers and Smith *supra.*

The modified insect cells may then be grown in an appropriate nutrient medium, which allows for stable maintenance of the plasmid(s) present in the modified insect host. Where the expression product gene is under inducible control, the host may be grown to high density, and expression induced. Alternatively, where expression is constitutive, the product will be continuously expressed into the medium and the nutrient medium must be continuously circulated, while removing the product of interest and augmenting depleted nutrients. The product may be purified by such techniques as chromatography, *eg.* HPLC, affinity chromatography, ion exchange chromatography, *etc.*; electrophoresis; density gradient centrifugation; solvent extraction, *etc.* As appropriate, the product may be further purified, as required, so as to remove substantially any insect proteins which are also present in the medium, so as to provide a product which is at least substantially free of host debris, eg. proteins, lipids and polysaccharides.

In order to obtain protein expression, recombinant host cells derived from the transformants are incubated under conditions which allow expression of the recombinant protein encoding sequence. These conditions will vary, dependent upon the host cell selected. However, the conditions are readily ascertainable to those of ordinary skill in the art, based upon what is known in the art.

iii. Plant Systems

There are many plant cell culture and whole plant genetic expression systems known in the art. Exemplary plant cellular genetic expression systems include those described in patents, such as: US 5,693,506; US 5,659,122; and US 5,608,143. Additional examples of genetic expression in plant cell culture has been described by Zenk, Phytochemistry 30:3861-3863 (1991). Descriptions of plant protein signal peptides may be found in addition to the references described above in Vaulcombe et al., Mol. Gen. Genet. 209:33-40 (1987); Chandler et al., Plant Molecular Biology 3: 407-418 (1984); Rogers, J. Biol. Chem. 260:3731-3738 (1985); Rothstein et al., Gene 55:353-356 (1987); Whittier et al., Nucleic Acids Research 15:25152535 (1987); Wirsel et al., Molecular Microbiology 3:3-14 (1989); Yu et al., Gene 122:247-253 (1992). A description of the regulation of plant gene expression by the phytohormone, gibberellic acid and secreted enzymes induced by gibberellic acid can be found in R.L. Jones and J. MacMillin, Gibberellins: in: Advanced Plant Physiology,. Malcolm B. Wilkins, ed., 1984 Pitman Publishing Limited, London, pp. 21-52. References that describe other metabolically-regulated genes: Sheen, Plant Cell, 2:1027-1038(1990); Maas et al., EMBO J. 9:3447-3452 (1990); Benkel and Hickey, Proc. Natl. Acad. Sci. 84:1337-1339 (1987).

Typically, using techniques known in the art, a desired polynucleotide sequence is inserted into an expression cassette comprising genetic regulatory elements designed for operation in plants. The expression cassette is inserted into a desired expression vector with companion sequences upstream and downstream from the expression cassette suitable for expression in a plant host. The companion sequences will be of plasmid or viral origin and provide necessary characteristics to the vector to permit the vectors to move DNA from an original cloning host, such as bacteria, to the desired plant host. Thebasic bacterial/plant vector construct will preferably provide a broad host range prokaryote replication origin; a prokaryote selectable marker; and, for Agrobacterium transformations, T DNA sequences for Agrobacterium-mediated transfer to plant chromosomes. Where the heterologous gene is not readily amenable to detection, the construct will preferably also have a selectable marker gene suitable for determining if a plant cell has been transformed. A general review of suitable markers, for example for the members of the grass family, is found in Wilmink and Dons, 1993, Plant Mol. Biol. Reptr, 11(2):165-185.

Sequences suitable for permitting integration of the heterologous sequence into the plant genome are also recommended. These might include transposon sequences and the like for homologous recombination as well as Ti sequences which permit random insertion of a heterologous expression cassette into a plant genome. Suitable prokaryote selectable markers include resistance toward antibiotics such as ampicillin or tetracycline. Other DNA sequences encoding additional functions may also be present in the vector, as is known in the art.

The nucleic acid molecules of the subject invention may be included into an expression cassette for expression of the protein(s) of interest. Usually, there will be only one expression cassette, although two or more are feasible. The recombinant expression cassette will contain in addition to the heterologous protein encoding sequence the following elements, a promoter region, plant 5' untranslated sequences, initiation codon depending upon whether or not the structural gene comes equipped with one, and a transcription and translation termination sequence. Unique restriction enzyme sites at the 5' and 3' ends of the cassette allow for easy insertion into a pre-existing vector.

A heterologous coding sequence may be for any protein relating to the present invention. The sequence encoding the protein of interest will encode a signal peptide which allows processing and translocation of the protein, as appropriate, and will usually lack any sequence which might result in the binding of the desired protein of the invention to a membrane. Since, for the most part, the transcriptional initiation region will be for a gene which is expressed and translocated during germination, by employing the signal peptide which provides for translocation, one may also provide for translocation of the protein of interest. In this way, the protein(s) of interest will be translocated from the cells in which they are expressed and may be efficiently harvested. Typically secretion in seeds are across the aleurone or scutellar epithelium layer into the endosperm of the seed. While it is not required that the protein be secreted from the cells in which the protein is produced, this facilitates the isolation and purification of the recombinant protein.

Since the ultimate expression of the desired gene product will be in a eucaryotic cell it is desirable to determine whether any portion of the cloned gene contains sequences which will be processed out as introns by the host's splicosome machinery. If so, site-directed mutagenesis of the "intron" region may be conducted to prevent losing a portion of the genetic message as a false intron code, Reed and Maniatis, Cell 41:95-105, 1985.

The vector can be microinjected directly into plant cells by use of micropipettes to mechanically transfer the recombinant DNA. Crossway, Mol. Gen. Genet, 202:179-185, 1985. The genetic material may also be transferred into the plant cell by using polyethylene glycol, Krens, et al., Nature, 296, 72-74, 1982. Another method of introduction of nucleic acid segments is high velocity ballistic penetration by small particles with the nucleic acid either within the matrix of small beads or particles, or on the surface, Klein, et al., Nature, 327, 70-73, 1987 and Knudsen and Muller, 1991, Planta, 185:330-336 teaching particle bombardment of barley endosperm to create transgenic barley. Yet another method of introduction would be fusion of protoplastswith other entities, either minicells, cells, lysosomes or other fusible lipidsurfaced bodies, Fraley, et al., Proc. Natl. Acad. Sci. USA, 79, 1859-1863, 1982.

The vector may also be introduced into the plant cells by electroporation. (Fromm et al., Proc. Natl Acad. Sci. USA 82:5824, 1985). In this technique, plant protoplasts are electroporated in the presence of plasmids containing the gene construct. Electrical impulses of high field strength reversibly permeabilize biomembranes allowing the introduction of the plasmids. Electroporated plant protoplasts reform the cell wall, divide, and form plant callus.

All plants from which protoplasts can be isolated and cultured to give whole regenerated plants can be transformed by the present invention so that whole plants are recovered which contain the transferred gene. It is known that practically all plants can be regenerated from cultured cells or tissues, including but not limited to all major species of sugarcane, sugar beet, cotton, fruit and other trees, legumes and vegetables. Some suitable plants include, for example, species from the genera *Fragaria, Lotus, Medicago, Onobrychis, Trifolium, Trigonella, Vigna, Citrus, Linum, Geranium, Manihot, Daucus, Arabidopsis, Brassica, Raphanus, Sinapis, Atropa, Capsicum, Datura, Hyoscyamus, Lycopersion, Nicotiana, Solanum, Petunia, Digitalis, Majorana, Cichorium, Helianthus, Lactuca, Bromus, Asparagus, Antirrhinum, Hererocallis, Nemesia, Pelargonium, Panicum, Pennisetum, Ranunculus, Senecio, Salpiglossis, Cucumis, Browaalia, Glycine, Lolium, Zea, Triticum, Sorghum,* and *Datura.*

Means for regeneration vary from species to species of plants, but generally a suspension of transformed protoplasts containing copies of the heterologous gene is first provided. Callus tissue is formed and shoots may be induced from callus and subsequently rooted. Alternatively, embryo formation can be induced from the protoplast suspension. These embryos germinate as natural embryos to form plants. The culture media will generally contain various amino acids and hormones, such as auxin and cytokinins. It is also advantageous to add glutamic acid and proline to the medium, especially for such species as corn and alfalfa. Shoots and roots normally develop simultaneously. Efficient regeneration will depend on the medium, on the genotype, and on the history of the culture. If these three variables are controlled, then regeneration is fully reproducible and repeatable.

In some plant cell culture systems, the desired protein of the invention may be excreted or alternatively, the protein may be extracted from the whole plant. Where the desired protein of the invention is secreted into the medium, it may be collected. Alternatively, the embryos and embryolesshalf seeds or other plant tissue may be mechanically disrupted to release any secreted protein between cells and tissues. The mixture may be suspended in a buffer solution to retrieve soluble proteins. Conventional protein isolation and purification methods will be then used to purify the recombinant protein. Parameters of time, temperature pH, oxygen, and volumes will be adjusted through routine methods to optimize expression and recovery of heterologous protein.

<u>iv. Bacterial Systems</u>

Bacterial expression techniques are known in the art. A bacterial promoter is any DNA sequence capable of binding bacterial RNA polymerase and initiating the downstream (3') transcription of a coding sequence (*eg.* structural gene) into mRNA. A promoter will have a transcription initiation region which is usually placed proximal to the 5' end of the coding sequence. This transcription initiation region usually includes an RNA polymerase binding site and a transcription initiation site. A bacterial promoter may also have a second domain called an operator, that may overlap an adjacent RNA polymerase binding site at which RNA synthesis begins. The operator permits negative regulated (inducible) transcription, as a gene repressor protein may bind the operator and thereby inhibit transcription of a specific gene. Constitutive expression may occur in the absence of negative regulatory elements, such as the operator. In addition, positive regulation may be achieved by a gene activator protein binding sequence, which, if present is usually proximal (5') to the RNA polymerase binding sequence. An example of a gene activator protein is the catabolite activator protein (CAP), which helps initiate transcription of the lac operon in Escherichia coli (*E. coli*) [Raibaud et al. (1984) Annu. Rev. Genet. 18:173]. Regulated expression may therefore be either positive or negative, thereby either enhancing or reducing transcription.

Sequences encoding metabolic pathway enzymes provide particularly useful promoter sequences. Examples include promoter sequences derived from sugar metabolizing enzymes, such as galactose, lactose (*lac*) [Chang et al. (1977) Nature 198:1056], and maltose. Additional examples include promoter sequences derived from biosynthetic enzymes such as tryptophan (*trp*) [Goeddel et al. (1980) Nuc. Acids Res. 8:4057; Yelverton et al. (1981) Nucl. Acids Res. 9:731; US patent 4,738,921; EP-A-0036776 and EP-A-0121775]. The g-laotamase (*bla*) promoter system [Weissmann (1981) "The cloning of interferon and other mistakes." In Interferon 3 (ed. I. Gresser)], bacteriophage lambda PL [Shimatake et al. (1981) Nature 292:128] and T5 [US patent 4,689,406] promoter systems also provide useful promoter sequences.

In addition, synthetic promoters which do not occur in nature also function as bacterial promoters. For example, transcription activation sequences of one bacterial or bacteriophage promoter may be joined with the operon sequences of another bacterial or bacteriophage promoter, creating a synthetic hybrid promoter [US patent 4,551,433]. For example, the *tac* promoter is a hybrid *trp-lac* promoter comprised of both *trp* promoter and *lac* operon sequences that is regulated by the *lac* repressor [Amann et al. (1983) Gene 25:167; de Boer et al. (1983) Proc. Natl. Acad. Sci. 80:21]. Furthermore, a bacterial promoter can include naturally occurring promoters of non-bacterial origin that have the ability to bind bacterial RNA polymerase and initiate transcription. A naturally occurring promoter of non-bacterial origin can also be coupled with a compatible RNA polymerase to produce high levels of expression of some genes in prokaryotes. The bacteriophage T7 RNA polymerase/promoter system is an example of a coupled promoter system [Studier et al. (1986) J. Mol. Biol. 189:113; Tabor et al. (1985) Proc Natl. Acad. Sci. 82:1074]. In addition, a hybrid promoter can also be comprised of a bacteriophage promoter and an *E. coli* operator region (EPO-A-0 267 851).

In addition to a functioning promoter sequence, an efficient ribosome binding site is also useful for the expression of foreign genes in prokaryotes. In *E. coli,* the ribosome binding site is called the Shine-Dalgarno (SD) sequence and includes an initiation codon (ATG) and a sequence 3-9 nucleotides in length located 3-11 nucleotides upstream of the initiation codon [Shineet al. (1975) Nature 254:34]. The SD sequence is thought to promote binding of mRNA to the ribosome by the pairing of bases between the SD sequence and the 3' and ofE. *coli* 16S rRNA [Steitz et al. (1979) "Genetic signals and nucleotide sequences in messenger RNA." In Biological Regulation and Development: Gene Expression (ed. R.F. Goldberger)]. To express eukaryotic genes and prokaryotic genes with weak ribosome-binding site [Sambrook et al. (1989) "Expression of cloned genes in Escherichia coli." In Molecular Cloning: A Laboratory Manual].

A DNA molecule may be expressed intracellularly. A promoter sequence may be directly linked with the DNA molecule, in which case the first amino acid at the N-terminus will always be a methionine, which is encoded by the ATG start codon. If desired, methionine at the N-terminus may be cleaved from the protein by *in vitro* incubation with cyanogen bromide or by either *in vivo* on *in vitro* incubation with a bacterial methionine N-terminal peptidase (EPO-A-0 219 237).

Fusion proteins provide an alternative to direct expression. Usually, a DNA sequence encoding the N-terminal portion of an endogenous bacterial protein, or other stable protein, is fused to the 5' end of heterologous coding sequences. Upon expression, this construct will provide a fusion of the tvo amino acid sequences. For example, the bacteriophage lambda cell gene can be linked at the 5' terminus of a foreign gene and expressed in bacteria. The resulting fusion protein preferably retains a site for a processing enzyme (factor Xa) to cleave the bacteriophage protein from the foreign gene [Nagai et al. (1984) Nature 309:810]. Fusion proteins can also be made with sequences from the *lac*Z [Jia et al. (1987) Gene 60:197], *trpE* [Allen et al. (1987) J. Biotechnol. 5:93; Makoff et al. (1989) J. Gen. Microbiol. 135:11], and *Chey* [EP-A-0 324 647] genes. The DNA sequence at the junction of the two amino acid sequences may or may not encode a cleavable site. Another example is a ubiquitin fusion protein. Such a fusion protein is made with the ubiquitin region that preferably retains a site for a processing enzyme (*eg*. ubiquitin specific processing-protease) to cleave the ubiquitin from the foreign protein. Through this method, native foreign protein can be isolated [Miller et al. (1989) Bio/ Technology 7:698].

Alternatively, foreign proteins can also be secreted from the cell by creating chimeric DNA molecules that encode a fusion protein comprised of a signal peptide sequence fragment that provides for secretion of the foreign protein in bacteria [US patent 4,336,336]. The signal sequence fragment usually encodes a signal peptide comprised of hydrophobic amino acids which direct the secretion of the protein from the cell. The protein is either secreted into the growth media (gram-positive bacteria) or into the periplasmic space, located between the inner and outer membrane of the cell (gram-negative bacteria). Preferably there are processing sites, which can be cleaved either *in vivo* or *in vitro* encoded between the signal peptide fragment and the foreign gene.

DNA encoding suitable signal sequences can be derived from genes for secreted bacterial proteins, such as the *E. coli* outer membrane protein gene (*ompA*) [Masui et al. (1983), in: Experimental Manipulation of Gene Expression; Ghrayeb et al. (1984) EMBO J. 3:2437] and the *E. coli* alkaline phosphatase signal sequence (*phoA*) [Oka et al. (1985) Proc. Natl. Acad. Sci. 82:7212]. As an additional example, the signal sequence of the alpha-amylase gene from various Bacillus strains can be used to secrete heterologous proteins from *B. subtilis* [Palva et al. (1982) Proc. Natl. Acad. Sci. USA 79:5582; EP-A-0 244 042].

Usually, transcription termination sequences recognized by bacteria are regulatory regions located 3' to the translation stop codon, and thus together with the promoter flank the coding sequence. These sequences direct the transcription of an mRNA which can be translated into the polypeptide encoded by the DNA. Transcription termination sequences frequently include DNA sequences of about 50 nucleotides capable of forming stem loop structures that aid in terminating transcription. Examples include transcription termination sequences derived from genes with strong promoters, such as the *trp* gene in *E. coli* as well as other biosynthetic genes.

Usually, the above described components, comprising a promoter, signal sequence (if desired), coding sequence of interest, and transcription termination sequence, are put together into expression constructs. Expression constructs are often maintained in a replicon, such as an extrachromosomal element (*eg*. plasmids) capable of stable maintenance in a host, such as bacteria. The replicon will have a replication system, thus allowing it to be maintained in a prokaryotic host either for expression or for cloning and amplification. In addition, a replicon may be either a high or low copy number plasmid. A high copy number plasmid will generally have a copy number ranging from about 5 to about 200, and usually about 10 to about 150. A host containing a high copy number plasmid will preferably contain at least about 10, and more preferably at least about 20 plasmids. Either a high or low copy number vector may be selected, depending upon the effect of the vector and the foreign protein on the host.

Alternatively, the expression constructs can be integrated into the bacterial genome with an integrating vector. Integrating vectors usually contain at least one sequence homologous to the bacterial chromosome that allows the vector to integrate. Integrations appear to result from recombinations between homologous DNA in the vector and the bacterial chromosome. For example, integrating vectors constructed with DNA from various Bacillus strains integrate into the Bacillus chromosome (EP-A- 0 127 328). Integrating vectors may also be comprised of bacteriophage or transposon sequences.

Usually, extrachromosomal and integrating expression constructs may contain selectable markers to allow for the selection of bacterial strains that have been transformed. Selectable markers can be expressed in the bacterial host and may include genes which render bacteria resistant to drugs such as ampicillin, chloramphenicol, erythromycin, kanamycin (neomycin), and tetracycline [Davies et al. (1978) Annu. Rev. Microbiol. 32:469].

Selectable markers may also include biosynthetic genes, such as those in the histidine, tryptophan, and leucine biosynthetic pathways.

Alternatively, some of the above described components can be put together in transformation vectors. Transformation vectors are usually comprised of a selectable market that is either maintained in a replicon or developed into an integrating vector, as described above.

Expression and transformation vectors, either extra-chromosomal replicons or integrating vectors, have been developed for transformation into many bacteria. For example, expression vectors have been developed for, *inter alia,* the following bacteria: Bacillus subtilis [Palva et al. (1982) Proc. Natl. Acad. Sci. USA 79:5582; EP-A-0 036 259 and EP-A-0 063 953; WO 84/04541], Escherichia coli [Shimatake et al. (1981) Nature 292:128; Amann et al. (1985) Gene 40: 183; Studier et al. (1986) J. Mol. Biol. 189:113; EP-A-0 036 776,EP-A-0 136 829 and EP-A-0 136 907], Streptococcus cremoris [Powell et al. (1988) Appl. Environ. Microbiol. 54:655]; Streptococcus lividans [Powell et al. (1988) Appl. Environ. Microbiol. 54:655], Streptomyces lividans [US patent 4,745,056].

Methods of introducing exogenous DNA into bacterial hosts are well-known in the art, and usually include either the transformation of bacteria treated with $CaCl_2$ or other agents, such as divalent cations and DMSO. DNA can also be introduced into bacterial cells by electroporation. Transformation procedures usually vary with the bacterial species to be transformed. See *eg.* [Masson et al. (1989) FEMS Microbiol. Lett. 60:273; Palva et al. (1982) Proc. Natl. Acad. Sci. USA 79:5582; EP-A-0 036 259 and EP-A-0 063 953; WO 84/04541, Bacillus], [Miller et al. (1988) Proc. Natl. Acad. Sci. 85:856; Wang et al. (1990) J. Bacteriol. 172:949, Campylobacter], [Cohen et al. (1973) Proc. Natl. Acad. Sci. 69: 2110; Dower et al. (1988) Nucleic Acids Res. 16:6127; Kushner (1978) "An improved method for transformation of Escherichia coli with ColEl-derived plasmids. In Genetic Engineering: Proceedings of the International Symposium on Genetic Engineering (eds. H.W. Boyer and S. Nicosia); Mandel et al. (1970) J. Mol. Biol. 53:159; Taketo (1988) Biochim. Biophys. Acta 949:318; Escherichia], [Chassy et al. (1987) FEMS Microbiol. Lett. 44:173 Lactobacillus]; [Fiedler et al. (1988) Anal. Biochem 170:38, Pseudomonas]; [Augustin et al. (1990) FEMS Microbiol. Lett. 66:203, Staphylococcus], [Barany et al. (1980) J. Bacteriol. 144:698; Harlander (1987) "Transformation of Streptococcus lactis by electroporation, in: Streptococcal Genetics (ed. J. Ferretti and R. Curtiss III); Perry et al. (1981) Infect. Immun. 32:1295; Powell et al. (1988) Appl. Environ. Microbiol. 54:655; Somkuti et al. (1987) Proc. 4th Evr. Cong. Biotechnology 1:412, Streptococcus].

v. Yeast Expression

Yeast expression systems are also known to one of ordinary skill in the art. A yeast promoter is any DNA sequence capable of binding yeast RNA polymerase and initiating the downstream (3') transcription of a coding sequence (*eg*. structural gene) into mRNA. A promoter will have a transcription initiation region which is usually placed proximal to the 5' end of the coding sequence. This transcription initiation region usually includes an RNA polymerase binding site (the "TATA Box") and a transcription initiation site. Ayeast promoter may also have a second domain called an upstream activator sequence (UAS), which, if present, is usually distal to the structural gene. The UAS permits regulated (inducible) expression. Constitutive expression occurs in the absence of a UAS. Regulated expression may be either positive or negative, thereby either enhancing or reducing transcription.

Yeast is a fermenting organism with an active metabolic pathway, therefore sequences encoding enzymes in the metabolic pathway provide particularly useful promoter sequences. Examples include alcohol dehydrogenase (ADH) (EP-A-0 284 044), enolase, glucokinase, glucose-6-phosphate isomerase, glyceraldehyde-3-phosphate-dehydrogenase (GAP or GAPDH), hexokinase, phosphofructokinase, 3-phosphoglycerate mutase, and pyruvate kinase (PyK) (EPO-A-0 329 203). The yeast *PHO5* gene, encoding acid phosphatase, also provides useful promoter sequences [Myanohara et al. (1983) Proc. Natl. Acad. Sci. USA 80:1].

In addition, synthetic promoters which do not occur in nature also function as yeast promoters. For example, UAS sequences of one yeast promoter may be joined with the transcription activation region of another yeast promoter, creating a synthetic hybrid promoter. Examples of such hybrid promoters include the ADH regulatory sequence linked to the GAP transcription activation region (US Patent Nos. 4,876,197 and 4,880,734). Other examples of hybrid promoters include promoters which consist of the regulatory sequences of either the*ADH2, GAL4, GAL10,* OR *PHO5* genes, combined with the transcriptional activation region of a glycolytic enzyme gene such as GAP or PyK (EP-A-0 164 556). Furthermore, a yeast promoter can include naturally occurring promoters of non-yeast origin that have the ability to bind yeast RNA polymerase and initiate transcription. Examples of such promoters include, *inter alia,* [Cohen et al. (1980) Proc. Natl. Acad. Sci. USA 77:1078; Henikoff et al. (1981) Nature 283:835; Hollenberg et al. (1981) Curr. Topics Microbiol. Immunol. 96:119; Hollenberg et al. (1979) "The Expression of Bacterial Antibiotic Resistance Genes in the Yeast Saccharomyces cerevisiae," in: Plasmids of Medical, Environmental and Commercial Importance (eds. K.N. Timmis and A. Puhler); Mercerau-Puigalon et al. (1980) Gene 11:163; Panthier et al. (1980) Curr. Genet. 2:109;].

A DNA molecule may be expressed intracellularly in yeast. A promoter sequence may be directly linked with the DNA molecule, in which case the first amino acid at the N-terminus of the recombinant protein will always be a methionine, which is encoded by the ATG start codon. If desired, methionine at the N-terminus may be cleaved from the protein by *in vitro* incubation with cyanogen bromide.

Fusion proteins provide an alternative for yeast expression systems, as well as in mammalian, baculovirus, and bacterial expression systems. Usually, a DNA sequence encoding the N-terminal portion of an endogenous yeast protein, or other stable protein, is fused to the 5' end of heterologous coding sequences. Upon expression, this construct will provide a fusion of the two amino acid sequences. For example, the yeast or human superoxide dismutase (SOD) gene, can be linked at the 5' terminus of a foreign gene and expressed in yeast. The DNA sequence at the junction of the two amino acid sequences may or may not encode a cleavable site. See *eg.* EP-A-0 196 056. Another example is a ubiquitin fusion protein. Such a fusion protein is made with the ubiquitin region that preferably retains a site for a processing enzyme (*eg.* ubiquitin-specific processing protease) to cleave the ubiquitin from the foreign protein. Through this method, therefore, native foreign protein can be isolated (*eg.* WO88/024066).

Alternatively, foreign proteins can also be secreted from the cell into the growth media by creating chimeric DNA molecules that encode a fusion protein comprised of a leader sequence fragment that provide for secretion in yeast of the foreign protein. Preferably, there are processing sites encoded between the leader fragment and the foreign gene that can be cleaved either *in vivo* or *in vitro.* The leader sequence fragment usually encodes a signal peptide comprised of hydrophobic amino acids which direct the secretion of the protein from the cell.

DNA encoding suitable signal sequences can be derived from genes for secreted yeast proteins, such as the yeast invertase gene (EP-A-0 012 873; JPO. 62,096,086) and the A-factor gene (US patent 4,588,684). Alternatively, leaders of non-yeast origin, such as an interferon leader, exist that also provide for secretion in yeast (EP-A-0 060 057).

A preferred class of secretion leaders are those that employ a fragment of the yeast alpha-factor gene, which contains both a "pre" signal sequence, and a "pro" region. The types of alpha-factor fragments that can be employed include the full-length pre-pro alpha factor leader (about 83 amino acid residues) as well as truncated alpha-factor leaders (usually about 25 to about 50 amino acid residues) (US Patents 4,546,083 and 4,870,008; EP-A-0 324 274). Additional leaders employing an alpha-factor leader fragment that provides for secretion include hybrid alpha-factor leaders made with a presequence of a first yeast, but a pro-region from a second yeast alphafactor. (*eg.* see WO 89/02463.)

Usually, transcription termination sequences recognized by yeast are regulatory regions located 3' to the translation stop codon, and thus together with the promoter flank the coding sequence. These sequences direct the transcription of an mRNA which can be translated into the polypeptide encoded by the DNA. Examples of transcription terminator sequence and other yeast-recognized termination sequences, such as those coding for glycolytic enzymes.

Usually, the above described components, comprising a promoter, leader (if desired), coding sequence of interest, and transcription termination sequence, are put together into expression constructs. Expression constructs are often maintained in a replicon, such as an extrachromosomal element (*eg.* plasmids) capable of stable maintenance in a host, such as yeast or bacteria. The replicon may have two replication systems, thus allowing it to be maintained, for example, in yeast for expression and in a prokaryotic host for cloning and amplification. Examples of such yeast-bacteria shuttle vectors include YEp24 [Botstein et al. (1979) Gene 8:17-24], pCl/l [Brake et al. (1984) Proc. Natl. Acad. Sci USA 81:4642-4646], and YRp17 [Stinchcomb et al. (1982) J. Mol. Biol. 158:157]. In addition, a replicon may be either a high or low copy number plasmid. A highcopy number plasmid will generally have a copy number ranging from about 5 to about 200, and usually about 10 to about 150. A host containing a high copy number plasmid will preferably have at least about 10, and more preferably at least about 20. Enter a high or low copy number vector may be selected, depending upon the effect of the vector and the foreign protein on the host. See *eg.* Brake *et al., supra.*

Alternatively, the expression constructs can be integrated into the yeast genome with an integrating vector. Integrating vectors usually contain at least one sequence homologous to a yeast chromosome that allows the vector to integrate, and preferably contain two homologous sequences flanking the expression construct. Integrations appear to result from recombinations between homologous DNA in the vector and the yeast chromosome [Orr-Weaver et al. (1983) Methods in Enzymol. 101:228-245]. An integrating vector may be directed to a specific locus in yeast by selecting the appropriate homologous sequence for inclusion in the vector. See Orr-Weaver *et al., supra.* One or more expression construct may integrate, possibly affecting levels of recombinant protein produced [Rine et al. (1983) Proc. Natl. Acad. Sci. USA 80:6750]. The chromosomal sequences included in the vector can occur either as a single segment in the vector, which results in the integra tion of the entire vector, or two segments homologous to adjacent segments in the chromosome and flanking the expression construct in the vector, which can result in the stable integration of only the expression construct.

Usually, extrachromosomal and integrating expression constructs may contain selectable markers to allow for the selection of yeast strains that have been transformed. Selectable markers may include biosynthetic genes that can be expressed in the yeast host, such as *ADE2, HIS4, LEU2, TRP1,* and *ALG7,* and the G418 resistance gene, which confer resistance in yeast cells to tunicamycin and G418, respectively. In addition, a suitable selectable marker may also provide yeast with the ability to grow in the presence of toxic compounds, such as metal. For example, the presence of *CUP1* allows yeast to grow in the presence of copper ions [Butt et al. (1987) Microbiol, Rev. 51:351].

Alternatively, some of the above described components can be put together into transformation vectors. Transformation vectors are usually comprised of a selectable marker that is either maintained in a replicon or developed into an integrating vector, as described above.

Expression and transformation vectors, either extrachromosomal replicons or integrating vectors, have been developed for transformation into many yeasts. For example, expression vectors have been developed for,*inter alia,* the following yeasts:Candida albicans [Kurtz, et al. (1986) Mol. Cell. Biol. 6:142], Candida maltosa [Kunze, et al. (1985) J. Basic Microbiol. 25:141]. Hansenula polymorpha [Gleeson, et al. (1986) J. Gen. Microbiol. 132:3459; Roggenkamp et al. (1986) Mol. Gen. Genet. 202:302], Kluyveromyces fragilis [Das, et al. (1984) J. Bacteriol. 158:1165], Kluyveromyces lactis [De Louvencourt et al. (1983) J. Bacteriol. 154:737; Van den Berg et al. (1990) Bio/Technology 8:135], Pichia guillerimondii [Kunze et al. (1985) J. Basic Microbiol. 25:141], Pichia pastoris [Cregg, et al. (1985) Mol. Cell. Biol. 5: 3376; US Patent Nos. 4,837,148 and 4,929,555], Saccharomyces cerevisiae [Hinnen et al. (1978) Proc. Natl. Acad. Sci. USA 75:1929; Ito et al. (1983) J. Bacteriol. 153:163], Schizosaccharomyces pombe [Beach and Nurse (1981) Nature 300:706], and Yarrowia lipolytica [Davidow, et al. (1985) Curr. Genet. 10:380471 Gaillardin, et al. (1985) Curr. Genet. 10:49].

Methods of introducing exogenous DNA into yeast hosts are well-known in the art, and usually include either the transformation of spheroplasts or of intact yeast cells treated with alkali cations. Transformation procedures usually vary with the yeast species to be transformed. See *eg.* [Kurtz et al. (1986) Mol. Cell. Biol. 6:142; Kunze et al. (1985) J. Basic Microbiol. 25:141; Candida]; [Gleeson et al. (1986) J. Gen. Microbiol. 132:3459; Roggenkamp et al. (1986) Mol. Gen. Genet. 202:302; Hansenula]; [Das et al. (1984) J. Bacteriol. 158:1165; De Louvencourt et al. (1983) J. Bacteriol. 154:1165; Van den Berg et al. (1990) Bio/Technology 8:135; Kluyveromyces]; [Cregg et al. (1985) Mol. Cell. Biol. 5: 3376; Kunze et al. (1985) J. Basic Microbiol. 25:141; US Patent Nos. 4,837,148 and 4,929,555; Pichia]; [Hinnenet al. (1978) Proc. Natl. Acad. Sci. USA 75;1929; Ito et al. (1983) J. Bacteriol. 153:163 Saccharomyces]; [Beach and Nurse (1981) Nature 300:706; Schizosaccharomyces]; [Davidow et al. (1985) Curr. Genet. 10:39; Gaillardin et al. (1985) Curr. Genet. 10:49; Yarrowia].

*Antibodies*

As used herein, the term "antibody" refers to a polypeptide or group of polypeptides composed of at least one antibody combining site. An "antibody combining site" is the three-dimensional binding space with an internal surface shape and charge distribution complementary to the features of an epitope of an antigen, which allows a binding of the antibody with the antigen. "Antibody" includes, for example, vertebrate antibodies, hybrid antibodies, chimeric antibodies, humanised antibodies, altered antibodies, univalent antibodies, Fab proteins, and single domain antibodies.

Antibodies against the proteins of the invention are useful for affinity chromatography, immunoassays, and distinguishing/identifying streptococcus proteins.

Antibodies to the proteins of the invention, both polyclonal and monoclonal, may be prepared by conventional methods. In general, the protein is first used to immunize a suitable animal, preferably a mouse, rat, rabbit or goat. Rabbits and goats are preferred for the preparation of polyclonal sera due to the volume of serum obtainable, and the availability of labeled anti-rabbit and anti-goat antibodies. Immunization is generally performed by mixing or emulsifying the protein in saline, preferably in an adjuvant such as Freund's complete adjuvant, and injecting the mixture or emulsion parenterally (generally subcutaneously or intramuscularly). A dose of 50-200 μg/injection is typically sufficient. Immunization is generally boosted 2-6 weeks later with one or more injections of the protein in saline, preferably using Freund's incomplete adjuvant. One may alternatively generate antibodies by in vitro immunization using methods known in the art, which for the purposes of this invention is considered equivalent to *in vivo* immunization. Polyclonal antisera is obtained by bleeding the immunized animal into a glass or plastic container, incubating the blood at 25°C for one hour, followed by incubating at 4°C for 2-18 hours. The serum is recovered by centrifugation (*eg.* 1,000g for 10 minutes). About 20-50 ml per bleed may be obtained from rabbits.

Monoclonal antibodies are prepared using the standard method of Kohler & Milstein [Nature (1975) 256:495-96], or a modification thereof. Typically, a mouse or rat is immunized as described above. However, rather than bleeding the animal to extract serum, the spleen (and optionally several large lymph nodes) is removed and dissociated into single cells. If desired, the spleen cells may be screened (after removal of nonspecifically adherent cells) by applying a cell suspension to a plate or well coated with the protein antigen. B-cells expressing membrane-bound immunoglobulin specific for the antigen bind to the plate, and are not rinsed away with the rest of the suspension. Resulting B-cells, or all dissociated spleen cells, are then induced to fuse with myeloma cells to form hybridomas, and are cultured in a selective medium (*eg.* hypoxanthine, aminopterin, thymidine medium, "HAT"). The resulting hybridomas are plated by limiting dilution, and are assayed for production of antibodies which bind specifically to the immunizing antigen (and which do not bind to unrelated antigens). The selected MAb-secreting hybridomas are then cultured either *in vitro* (*eg.* in tissue culture bottles or hollow fiber reactors), or *in vivo* (as ascites in mice).

If desired, the antibodies (whether polyclonal or monoclonal) may be labeled using conventional techniques. Suitable labels include fluorophores, chromophores, radioactive atoms (particularly$^{32}$P and $^{125}$I), electron-dense reagents, enzymes, and ligands having specific binding partners. Enzymes are typically detected by their activity. For example, horseradish peroxidase is usually detected by its ability to convert 3,3',5,5'-tetramethylbenzidine (TMB) to a blue pigment, quantifiable with a spectrophotometer. "Specific binding partner" refers to a protein capable of binding a ligand molecule with high specificity, as for example in the case of an antigen and a monoclonal antibody specific therefor. Other specific binding partners include biotin and avidin or streptavidin, IgG and protein A, and the numerous receptor-ligand couples known in the art. It should be understood that the above description is not meant to categorize the various labels into distinct classes, as the same label may serve in several different modes. For example, $^{125}$I may serve as a radioactive label or as an electron-dense reagent. HRP may serve as enzyme or as antigen for a MAb. Further, one may combine various labels for desired effect. For example, MAbs and avidin also require labels in the practice of this invention: thus, one might label a MAb with biotin, and detect its presence with avidin labeled with $^{125}$I, or with an anti-biotin MAb labeled with HRP. Other permutations and possibilities will be readily apparent to those of ordinary skill in the art, and are considered as equivalents within the scope of the instant invention.

## *Pharmaceutical Compositions*

Pharmaceutical compositions can comprise either polypeptides, antibodies, or nucleic acid of the invention. The pharmaceutical compositions will comprise a therapeutically effective amount of either polypeptides, antibodies, or polynucleotides of the claimed invention.

The term "therapeutically effective amount" as used herein refers to an amount of a therapeutic agent to treat, ameliorate, or prevent a desired disease or condition, or to exhibit a detectable therapeutic orpreventative effect. The effect can be detected by, for example, chemical markers or antigen levels. Therapeutic effects also include reduction in physical symptoms, such as decreased body temperature. The precise effective amount for a subject will depend upon the subject's size and health, the nature and extent of the condition, and the therapeutics or combination of therapeutics selected for administration. Thus, it is not useful to specify an exact effective amount in advance. However, the effective amount for a given situation can be determined by routine experimentation and is within the judgement of the clinician.

For purposes of the present invention, an effective dose will be from about 0.01 mg/ kg to 50 mg/kg or 0.05 mg/kg to about 10 mg/kg of the DNA constructs in the individual to which it is administered.

A pharmaceutical composition can also contain a pharmaceutically acceptable carrier. The term "pharmaceutically acceptable carrier" refers to a carrier for administration of a therapeutic agent, such as antibodies or a polypeptide, genes, and other therapeutic agents. The term refers to any pharmaceutical carrier that does not itself induce the production of antibodies harmful to the individual receiving the composition, and which may be administered without undue toxicity. Suitable carriers may be large, slowly metabolized macromolecules such as proteins, polysaccharides, polylactic acids, polyglycolic acids, polymeric amino acids, amino acid copolymers, and inactive virus particles. Such carriers are well known to those of ordinary skill in the art.

Pharmaceutically acceptable salts can be used therein, for example, mineral acid salts such as hydrochlorides, hydrobromides, phosphates, sulfates, and the like; and the salts of organic acids such as acetates, propionates, malonates, benzoates, and the like. A thorough discussion of pharmaceutically acceptable excipients is available in Remington's Pharmaceutical Sciences (Mack Pub. Co., N.J. 1991).

Pharmaceutically acceptable carriers in therapeutic compositions may contain liquids such as water, saline, glycerol and ethanol. Additionally, auxiliary substances, such as wetting or emulsifying agents, pH buffering substances, and the like, may be present in such vehicles. Typically, the therapeutic compositions are prepared as injectables, either as liquid solutions or suspensions; solid forms suitable for solution in, or suspension in, liquid vehicles prior to injection may also be prepared. Liposomes are included within the definition of a pharmaceutically acceptable carrier.

## *Delivery Methods*

Once formulated, the compositions of the invention can be administered directly to the subject. The subjects to be treated can be animals; in particular, human subjects can be treated.

Direct delivery of the compositions will generally be accomplished by injection, either subcutaneously, intraperitoneally, intravenously or intramuscularly or delivered to the interstitial space of a tissue. The compositions can also be administered into a lesion. Other modes of administration include oral and pulmonary administration, suppositories, and transdermal or transcutaneous applications (*eg.* see WO98/20734), needles, and gene guns or hyposprays. Dosage treatment may be a single dose schedule or a multiple dose schedule.

## *Vaccines*

Vaccines according to the invention may either be prophylactic (*ie.* to prevent infection) or therapeutic (*ie.* to treat disease after infection).

Such vaccines comprise immunising antigen(s), immunogen(s), polypeptide(s), protein(s) or nucleic acid, usually in combination with "pharmaceutically acceptable carriers," which include any carrier that does not itself induce the production of antibodies harmful to the individual receiving the composition. Suitable carriers are typically large, slowly metabolized macromolecules such as proteins, polysaccharides, polylactic acids, polyglycolic acids, polymeric amino acids, amino acid copolymers, lipid aggregates (such as oil droplets or liposomes), and inactive virus particles. Such carriers are well known to those of ordinary skill in the art. Additionally, these carriers may function as immunostimulating agents ("adjuvants"). Furthermore, the antigen or immunogen may be conjugated to a bacterial toxoid, such as a toxoid from diphtheria, tetanus, cholera, *H. pylori, etc.* pathogens.

Preferred adjuvants to enhance effectiveness of the composition include, but are not limited to: (1) aluminum salts (alum), such as aluminum hydroxide, aluminum phosphate, aluminum sulfate, etc; (2) oil-in-water emulsion formulations (with or without other specific immunostimulating agents such as muramyl peptides (see below) or bacterial cell wall components), such as for example (a) MF59™ (WO 90/14837; Chapter 10 in Vaccine design: the subunit and adjuvant approach, eds. Powell & Newman, Plenum Press 1995), containing 5% Squalene, 0.5% Tween 80, and 0.5% Span 85 (optionally containing various amounts of MTP-PE (see below), although not required) formulated into submicron particles using a microfluidizer such as Model 110Y microfluidizer (Microfluidics, Newton, MA), (b) SAF, containing 10% Squalane, 0.4% Tween 80, 5% pluronic-blocked polymer L121, and thr-MDP (see below) either microfluidized into a submicron emulsion or vortexed to generate a larger particle size emulsion, and (c) Ribi™ adjuvant system (RAS), (Ribi Immunochem, Hamilton, MT) containing 2% Squalene, 0.2% Tween 80, and one or more bacterial cell wall components from the group consisting of monophosphorylipid A (MPL), trehalose dimycolate (TDM), and cell wall skeleton (CWS), preferably MPL + CWS (Detox™); (3) saponin adjuvants, such as Stimulon™ (Cambridge Bioscience, Worcester, MA) may be used or particles generated therefrom such as ISCOMs (immunostimulating complexes); (4) Complete Freund's Adjuvant (CFA) and Incomplete Freund's Adjuvant (IFA); (5) cytokines, such as interleukins (*eg.* IL-1, IL-2, IL-4, IL-5, IL-6, IL-7, IL-12, *etc.),* interferons (*eg.* gamma interferon), macrophage colony stimulating factor (M-CSF), tumor necrosis factor (TNF), etc; and (6) other substances that act as immunostimulating agents to enhance the effectiveness of the composition. Alum and MF59™ are preferred.

As mentioned above, muramyl peptides include, but are not limited to, N-acetyl-muramyl-L-threonyl-D-isoglutamine (thr-MDP), N-acetyl-normuramyl-L-alanyl-D-isoglutamine (nor-MDP), N-acetylmuramyl-L-alanyl-D-isoglutaminyl-L-alanine-2-(1'-2'-dipalmitoyl-*sn*-glycero-3-hydroxyphosphoryloxy)-ethylamine (MTP-PE), *etc.*

The immunogenic compositions (*eg.* the immunising antigen/immunogen/polypeptide/protein/ nucleic acid, pharmaceutically acceptable carrier, and adjuvant) typically will contain diluents, such as water, saline, glycerol, ethanol, etc. Additionally, auxiliary substances, such as wetting or emulsifying agents, pH buffering substances, and the like, may be present in such vehicles.

Typically, the immunogenic compositions are prepared as injectables, either as liquid solutions or suspensions; solid forms suitable for solution in, or suspension in, liquid vehicles prior to injection may also be prepared. The preparation also may be emulsified or encapsulated in liposomes for enhanced adjuvant effect, as discussed above under pharmaceutically acceptable carriers.

Immunogenic compositions used as vaccines comprise an immunologically effective amount of the antigenic or immunogenic polypeptides, as well as any other of the above-mentioned components, as needed. By "immunologically effective amount", it is meant that the administration of that amount to an individual, either in a single dose or as part of a series, is effective for treatment or prevention. This amount varies depending upon the health and physical condition of the individual to be treated, the taxonomic group of individual to be treated (*eg.* nonhuman primate, primate, *etc.),* the capacity of the individual's immune system to synthesize antibodies, the degree of protection desired, the formulation of the vaccine, the treating doctor's assessment of the medical situation, and other relevant factors. It is expected that the amount will fall in a relatively broad range that can be determined through routine trials.

The immunogenic compositions are conventionally admiristered parenterally, *eg.* by injection, either subcutaneously, intramuscularly, or transdermally/transcutaneously (*eg.* WO98/20734). Additional formulations suitable for other modes of administration include oral and pulmonary formulations, suppositories, and transdermal applications. Dosage treatment may be a single dose schedule or a multiple dose schedule. The vaccine may be administered in conjunction with other immunoregulatory agents.

As an alternative to protein-based vaccines, DNA vaccination may be used [*eg.* Robinson & Torres (1997) Seminars in Immunol 9:271-283; Donnelly et al. (1997) Annu Rev Immunol 15:617-648; later herein].

*Gene Delivery Vehicles*

Gene therapy vehicles for delivery of constructs including a coding sequence of a therapeutic of the invention, to be delivered to the mammal for expression in the mammal, can be administered either locally or systemically. These constructs can utilize viral or non-viral vector approaches in *in vivo* or *ex vivo* modality. Expression of such coding sequence can be induced using endogenous mammalian or heterologous promoters. Expression of the coding sequence in vivo can be either constitutive or regulated.

The invention includes gene delivery vehicles capable of expressing the contemplated nucleic acid sequences. The gene delivery vehicle is preferably a viral vector and, more preferably, a retroviral, adenoviral, adeno-associated viral (AAV), herpes viral, or alphavirus vector. The viral vector can also be an astrovirus, coronavirus, orthomyxovirus, papovavirus, paramyxovirus, parvovirus, picornavirus, poxvirus, or togavirus viral vector. See generally, Jolly (1994) Cancer Gene Therapy 1:51-64; Kimura (1994) Human Gene Therapy 5:845-852; Connelly (1995) Human Gene Therapy 6:185-193; and Kaplitt (1994) Nature Genetics 6:148-153.

Retroviral vectors are well known in the art and we contemplate that any retroviral gene therapy vector is employable in the invention, including B, C and D type retroviruses, xenotropic retroviruses (for example, NZB-X1, NZB-X2 and NZB9-1 (see O'Neill (1985) J. Virol. 53:160) polytropic retroviruses *eg.* MCF and MCF-MLV (see Kelly (1983) J. Virol. 45:291), spumaviruses and lentiviruses. See RNA Tumor Viruses, Second Edition, Cold Spring Harbor Laboratory, 1985.

Portions of the retroviral gene therapy vector may be derived from different retroviruses. For example, retrovector LTRs may be derived from a Murine Sarcoma Virus, a tRNA binding site from a Rous Sarcoma Virus, a packaging signal from a Murine Leukemia Virus, and an origin of second strand synthesis from an Avian Leukosis Virus.

These recombinant retroviral vectors may be used to generate transduction competent retroviral vector particles by introducing them into appropriate packaging cell lines (see US patent 5,591,624). Retrovirus vectorscan be constructed for site-specific integration into host cell DNA by incorporation of a chimeric integrase enzyme into the retroviral particle (see WO96/37626). It is preferable that the recombinant viral vector is a replication defective recombinant virus.

Packaging cell lines suitable for use with the above-described retrovirus vectors are well known in the art, are readily prepared (see WO95/30763 and WO92/05266), and can be used to create producer cell lines (also termed vector cell lines or "VCLs") for the production of recombinant vector particles. Preferably, the packaging cell lines are made from human parent cells (*eg.* HT1080 cells) or mink parent cell lines, which eliminates inactivation in human serum.

Preferred retroviruses for the construction of retroviral gene therapy vectors include Avian Leukosis Virus, Bovine Leukemia, Virus, Murine Leukemia Virus, Mink-Cell Focus-Inducing Virus, Murine Sarcoma Virus, Reticuloendotheliosis Virus and Rous Sarcoma Virus. Particularly preferred Murine Leukemia Viruses include 4070A and 1504A (Hartley and Rowe (1976) J Virol 19:19-25), Abelson (ATCC No. VR-999), Friend (ATCC No. VR-245), Graffi, Gross (ATCC Nol VR-590), Kirsten, Harvey Sarcoma Virus and Rauscher (ATCC No. VR-998) and Moloney Murine Leukemia Virus (ATCC No. VR-190). Such retroviruses may be obtained from depositories or collections such as the American Type Culture Collection ("ATCC") in Rockville, Maryland or isolated from known sources using commonly available techniques.

Exemplary known retroviral gene therapy vectors employable in this invention include those described in patent applications GB2200651, EP0415731, EP0345242, EP0334301, WO89/02468; WO89/05349, WO89/09271, WO90/02806, WO90/07936, WO94/03622, WO93/25698, WO93/25234, WO93/11230, WO93/10218, WO91/02805, WO91/02825, WO95/07994, US 5,219,740, US 4,405,712, US 4,861,719, US 4,980,289, US 4,777,127, US 5,591,624.

See also Vile (1993) Cancer Res 53:3860-3864; Vile (1993) Cancer Res 53:962-967; Ram (1993) Cancer Res 53 (1993) 83-88; Takamiya (1992) J Neurosci Res 33:493-503; Baba (1993) J Neurosurg 79:729-735; Mann (1983) Cell 33:153; Cane (1984) Proc Natl Acad Sci 81:6349; and Miller (1990) Human Gene Therapy 1.

Human adenoviral gene therapy vectors are also known in the art and employable in this invention. See, for example, Berkner (1988) Biotechniques 6:616 and Rosenfeld (1991) Science 252:431, and WO93/07283, WO93/06223, and WO93/07282. Exemplary known adenoviral gene therapy vectors employable in this invention include those described in the above referenced documents and in WO94/12649, WO93/03769, WO93/19191, WO94/28938, WO95/11984, WO95/00655, WO95/27071, WO95/29993, WO95/34671, WO96/05320, WO94/08026, WO94/11506, WO93/06223, WO94/24299, WO95/14102, WO95/24297, WO95/02697, WO94/28152, WO94/24299, WO95/09241, WO95/25807, WO95/05835, WO94/18922 and WO95/09654. Alternatively, administration of DNA linked to killed adenovirus as described in Curiel (1992) Hum. Gene Ther. 3:147-154 may be employed. The gene delivery vehicles of the invention also include adenovirus associated virus (AAV) vectors. Leading and preferred examples of such vectors for use in this invention are the AAV-2 based vectors disclosed in Srivastava, WO93/09239. Most preferred AAV vectors comprise the two AAV inverted terminal repeats in which the native D-sequences are modified by substitution of nucleotides, such that at least 5 native nucleotides and up to 18 native nucleotides, preferably at least 10 native nucleotides up to 18 native nucleotides, most preferably 10 rative nucleotides are retained and the remaining nucleotides of the D-sequence are deleted or replaced with non-native nucleotides. The native D-sequences of the AAV inverted terminal repeats are sequences of 20 consecutive nucleotides in each AAV inverted terminal repeat (*ie.* there is one sequence at each end) which are not involved in HP formation. The non-native replacement nucleotide may be any nucleotide other than the nucleotide found in the native D-sequence in the same position. Other employable exemplary AAV vectors are pWP-19, pWN-1, both of which are disclosed in Nahreini (1993) Gene 124:257-262. Another example of such an AAV vector is psub201 (see Samulski (1987) J. Virol. 61:3096). Another exemplary AAV vector is the Double-D ITR vector. Construction of the Double-D ITR vector is disclosed in US Patent 5,478,745. Still other vectors are those disclosed in Carter US Patent 4,797,368 and Muzyczka US Patent 5,139,941, Chartejee US Patent 5,474,935, and Kotin WO94/288157. Yet a further example of an AAV vector employable in this invention is SSV9AFABTKneo, which contains the AFP enhancer and albumin promoter and directs expression predominantly in the liver. Its structure and construction are disclosed in Su (1996) Human Gene Therapy 7:463-470. Additional AAV gene therapy vectors are described in US 5,354,678, US 5,173,414, US 5,139,941, and US 5,252,479.

The gene therapy vectors of the invention also include herpes vectors. Leading and preferred examples are herpes simplex virus vectors containing a sequence encoding a thymidine kinase polypeptide such as those disclosed in US 5,288,641 and EP0176170 (Roizman). Additional exemplary herpes simplex virus vectors include HFEM/ICP6-LacZ disclosed in WO95/04139 (Wistar Institute), pHSVlac described in Geller (1988) Science 241:1667-1669 and in WO90/09441 and WO92/07945, HSV Us3::pgGlacZ described in Fink (1992) Human Gene Therapy 3:11-19 and HSV 7134, 2 RH 105 and GAL4 described in EP 0453242 (Breakefield), and those deposited with the ATCC with accession numbers VR-977 and VR-260.

Also contemplated are alpha virus gene therapy vectors that can be employed in this invention. Preferred alpha virus vectors are Sindbis viruses vectors. Togaviruses, Semliki Forest virus (ATCC VR67; ATCC VR-1247), Middleberg virus (ATCC VR-370), Ross River virus (ATCC VR-373; ATCC VR-1246), Venezuelan equine encephalitis virus (ATCC VR923; ATCC VR-1250; ATCC VR-1249; ATCC VR-532), and those described in US patents 5,091,309, 5,217,879, and WO92/10578. More particularly, those alpha virus vectors described in US Serial No. 08/405,627, filed March 15, 1995,WO94/21792, WO92/10578, WO95/07994, US 5,091,309 and US 5,217,879 are employable. Such alpha viruses may be obtained from depositories or collections such as the ATCC in Rockville, Maryland or isolated from known sources using commonly available techniques. Preferably, alphavirus vectors with reduced cytotoxicity are used (see USSN 08/679640).

DNA vector systems such as eukaryotic layered expression systems are also useful for expressing the nucleic acids of the invention. See WO95/07994 for a detailed description of eukaryotic layered expression systems.

Preferably, the eukaryotic layered expression systems of the invention are derived from alphavirus vectors and most preferably from Sindbis viral vectors.

Other viral vectors suitable for use in the present invention include those derived from poliovirus, for example ATCC VR-58 and those described in Evans, Nature 339 (1989) 385 and Sabin (1973) *J. Biol. Standardization* 1:115; rhinovirus, for example ATCC VR-1110 and those described in Arnold (1990)J Cell Biochem L401; pox viruses such as canary pox virus or vaccinia virus, for example ATCC VR-111 and ATCC VR-2010 and those described in Fisher-Hoch (1989) Proc Natl Acad Sci 86:317; Flexner (1989) Ann NY Acad Sci 569:86, Flexner (1990) Vaccine 8:17; in US 4,603,112 and US 4,769,330 and WO89/01973; SV40 virus, for example ATCC VR-305 and those described in Mulligan (1979) Nature 277:108 and Madzak (1992) J Gen Virol 73:1533; influenza virus, for example ATCC VR-797 and recombinant influenza viruses made employing reverse genetics techniques as described in US 5,166,057 and in Enami (1990) Proc Natl Acad Sci 87:3802-3805; Enami & Palese (1991) J Virol 65:2711-2713 and Luytjes (1989) Cell 59:110, (see also McMichael (1983) NEJ Med 309:13, and Yap (1978) Nature 273:238 and Nature (1979) 277:108); human immunodeficiency virus as described in EP-0386882 and in Buchschacher (1992) J. Virol. 66:2731; measles virus, for example ATCC VR-67 and VR-1247 and those described in EP-0440219; Aura virus, for example ATCC VR-368; Bebaru virus, for example ATCC VR-600 and ATCC VR-1240; Cabassou virus, for example ATCC VR-922; Chikungunya virus, for example ATCC VR-64 and ATCC VR-1241; Fort Morgan Virus, for example ATCC VR-924; Getah virus, for example ATCC VR-369 and ATCC VR-1243; Kyzylagach virus, for example ATCC VR-927; Mayaro virus, for example ATCC VR-66; Mucambo virus, for example ATCC VR-580 and ATCC VR-1244; Ndumu virus, for example ATCC VR-371; Pixuna virus, for example ATCC VR-372 and ATCC VR-1245; Tonate virus, for example ATCC VR-925; Triniti virus, for example ATCC VR-469; Una virus, for example ATCC VR-374; Whataroa virus, for example ATCC VR-926; Y-62-33 virus, for example ATCC VR-375; O'Nyong virus, Eastern encephalitis virus, for example ATCC VR65 and ATCC VR-1242; Western encephalitis virus, for example ATCC VR-70, ATCC VR-1251, ATCC VR-622 and ATCC VR-1252; and coronavirus, for example ATCC VR-740 and those described in Hamre (1966) Proc Soc Exp Biol Med 121:190.

Delivery of the compositions of this invention into cells is not limited to the above mentioned viral vectors. Other delivery methods and media may be employed such as, for example, nucleic acid expression vectors, polycationic condensed DNA linked or unlinked to killed adenovirus alone, for example see US Serial No. 08/366,787, filed December 30, 1994 and Curiel (1992) Hum Gene Ther 3:147-154 ligand linked DNA, for example see Wu (1989) J Biol Chem 264: 16985-16987, eucaryotic cell delivery vehicles cells, for example see US Serial No.08/240,030, filed May 9, 1994, and US Serial No. 08/404,796, deposition of photopolymerized hydrogel materials, hand-held gene transfer particle gun, as described in US Patent 5,149,655, ionizing radiation as described in US5,206,152 and in WO92/11033 nucleic charge neutralization or fusion with cell membranes. Additional approaches are described in Philip (1994)Mol Cell Biol 14: 2411-2418 and in Woffendin (1994)Proc Natl Acad Sci 91:1581-1585.

Particle mediated gene transfer may be employed, for example see US Serial No. 60/023,867. Briefly, the sequence can be inserted into conventional vectors that contain conventional control sequences for high level expression, and then incubated with synthetic gene transfer molecules such as polymeric DNA-binding cations like polylysine, protamine, and albumin, linked to cell targeting ligands such as asialoorosomucoid, as described in Wu & Wu (1987) J. Biol. Chem. 262:4429-4432, insulin as described in Hucked (1990) Biochem Pharmacol 40:253-263, galactose as described in Plank (1992) Bioconjugate Chem 3:533-539, lactose or transferrin.

Naked DNA may also be employed. Exemplary naked DNA introduction methods are described in WO 90/11092 and US 5,580,859. Uptake efficiency may be improved using biodegradable latex beads. DNA coated latex beads are efficiently transported into cells after endocytosis initiation by the beads. The method may be improved further by treatment of the beads to increase hydrophobicity and hereby facilitate disruption of the endosome and release of the DNA into the cytoplasm.

Liposomes that can act as gene delivery vehicles are described in US 5,422,120, WO95/13796, WO94/23697, WO91/14445 and EP-524,968. As described in USSN. 60/023,867, on non-viral delivery, the nucleic acid sequences encoding a polypeptide can be inserted into conventional vectors that contain conventional control sequences for high level expression, and then be incubated with synthetic gene transfer molecules such as polymeric DNA-binding cations like polylysine, protamine, and albumin, linked to cell targeting ligands such as asialoorosomucoid, insulin, galactose, lactose, or transferrin. Other delivery systems include the use of liposomes to encapsulate DNA comprising the gene under the control of a variety of tissue-specific or ubiquitously-active promoters. Further non-viral delivery suitable for use includes mechanical delivery systems such as the approach described in Woffendin et al (1994) Proc. Natl. Acad. Sci. USA 91(24):11581-11585. Moreover, the coding sequence and the product of expression of such can be delivered through deposition of photopolymerized hydrogel materials. Other conventional methods for gene delivery that can be used for delivery of the coding sequence include, for example, use of hand-held gene transfer particle gun, as described in US 5,149,655; use of ionizing radiation for activating transferred gene, as described in US 5,206,152 and WO92/11033

Exemplary liposome and polycationic gene delivery vehicles are those described in US 5,422,120 and 4,762,915; in WO 95/13796; WO94/23697; and WO91/14445; in EP-0524968; and in Stryer, Biochemistry, pages 236-240 (1975) W.H. Freeman, San Francisco; Szoka (1980) Biochem Biophys Acta 600:1; Bayer (1979) Biochem Biophys Acta 550:464; Rivnay (1987) Meth Enzymol 149:119; Wang (1987) Proc Natl Acad Sci 84:7851; Plant (1989) Anal Biochem 176:420.

A polynucleotide composition can comprises therapeutically effective amount of a gene therapy vehicle, as the term is defined above. For purposes of the present invention, an effective dose will be from about 0.01 mg/ kg to 50 mg/kg or 0.05 mg/kg to about 10 mg/kg of the DNA constructs in the individual to which it is administered.

<u>*Delivery Methods*</u>

Once formulated, the polynucleotide compositions of the invention can be administered (1) directly to the subject; (2) delivered *ex vivo,* to cells derived from the subject; or (3) *in vitro* for expression of recombinant proteins. The subjects to be treated can be mammals or birds. Also, human subjects can be treated.

Direct delivery of the compositions will generally be accomplished by injection, either subcutaneously, intraperitoneally, intravenously or intramuscularly or delivered to the interstitial space of a tissue. The compositions can also be administered into a lesion. Other modes of administration include oral and pulmonary administration, suppositories, and transdermal or transcutaneous applications (*eg.* see WO98/20734), needles, and gene guns or hyposprays. Dosage treatment may be a single dose schedule or a multiple dose schedule.

Methods for the *ex vivo* delivery and reimplantation of transformed cells into a subject are known in the art and described in *eg.* WO93/14778. Examples of cells useful in ex vivo applications include, for example, stem cells, particularly hematopoetic, lymph cells, macrophages, dendritic cells, or tumor cells.

Generally, delivery of nucleic acids for both *ex vivo* and *in vitro* applications can be accomplished by the following procedures, for example, dextran-mediated transfection, calcium phosphate precipitation, polybrene mediated transfection, protoplast fusion, electroporation, encapsulation of the polynucleotide(s) in liposomes, and direct microinjection of the DNA into nuclei, all well known in the art.

*Polynucleotide and polypeptide pharmaceutical compositions*

In addition to the pharmaceutically acceptable carriers and salts described above, the following additional agents can be used with polynucleotide and/or polypeptide compositions.

A.Polypeptides

One example are polypeptides which include, without limitation: asioloorosomucoid (ASOR); transferrin; asialoglycoproteins; antibodies; antibody fragments; ferritin; interleukins; interferons, granulocyte, macrophage colony stimulating factor (GM-CSF), granulocyte colony stimulating factor (G-CSF), macrophage colony stimulating factor (M-CSF), stem cell factor and erythropoietin. Viral antigens, such as envelope proteins, can also be used. Also, proteins from other invasive organisms, such as the 17 amino acid peptide from the circumsporozoite protein of plasmodium falciparum known as RII.

B.Hormones, Vitamins, *etc.*

Other groups that can be included are, for example: hormones, steroids, androgens, estrogens, thyroid hormone, or vitamins, folic acid.

C.Polyalkylenes, Polysaccharides, *etc.*

Also, polyalkylene glycol can be included with the desired polynucleotides/polypeptides. In a preferred embodiment, the polyalkylene glycol is polyethlylene glycol. In addition, mono, di-, or polysaccharides can be included. In a preferred embodiment of this aspect, the polysaccharide is dextran or DEAEdextran. Also, chitosan and poly(lactide-co-glycolide)

D.Lipids, and Liposomes

The desired polynucleotide/polypeptide can also be encapsulated in lipids or packaged in liposomes prior to delivery to the subject or to cells derived therefrom.

Lipid encapsulation is generally accomplished using liposomes which are able to stably bind or entrap and retain nucleic acid. The ratio of condensed polynucleotide to lipid preparation can vary but will generally be around 1:1 (mg DNA:micromoles lipid), or more of lipid. For a review of the use of liposomes as carriers for delivery of nucleic acids, see, Hug and Sleight (1991) Biochim. Biophys. Acta. 1097:1-17; Straubinger (1983) Meth. Enzymol. 101:512-527.

Liposomal preparations for use in the present invention include cationic (positively charged), anionic (negatively charged) and neutral preparations. Cationic liposomes have been shown to mediate intracellular delivery of plasmid DNA (Felgner (1987) Proc. Natl. Acad. Sci. USA 84:7413-7416); mRNA (Malone (1989) Proc. Natl. Acad. Sci. USA 86: 6077-6081); and purified transcription factors (Debs (1990) J. Biol. Chem. 265:10189-10192), in functional form.

Cationic liposomes are readily available. For example, N[1-2,3-dioleyloxy)propyl]-N,N,N-triethylammonium (DOTMA) liposomes are available under the trademark Lipofectin, from GIBCO BRL, Grand Island, NY. (See, also, Felgner *supra*). Other commercially available liposomes include transfectace (DDAB/DOPE) and DOTAP/DOPE (Boerhinger). Other cationic liposomes can be prepared from readily available materials using techniques well known in the art. See, *eg.* Szoka (1978) Proc. Natl. Acad. Sci. USA 75:4194-4198; WO90/11092 for a description of the synthesis of DOTAP (1,2-bis(oleoyloxy)-3-(trimethylammonio)propane) liposomes.

Similarly, anionic and neutral liposomes are readily available, such as from Avanti Polar Lipids (Birmingham, AL), or can be easily prepared using readily available materials. Such materials include phosphatidyl choline, cholesterol, phosphatidyl ethanolamine, dioleoylphosphatidyl choline (DOPC), dioleoylphosphatidyl glycerol (DOPG), dioleoylphoshatidyl ethanolamine (DOPE), among others. These materials can also be mixed with he DOTMA and DOTAP starting materials in appropriate ratios. Methods for making liposomes using these materials are well known in the art.

The liposomes can comprise multilammelar vesicles (MLVs), small unilamellar vesicles (SUVs), or large unilamellar vesicles (LUVs). The various liposome-nucleic acid complexes are prepared using methods known in the art. See *eg.* Straubinger (1983) Meth. Immunol. 101:512-527; Szoka (1978) Proc. Natl. Acad. Sci. USA 75:4194-4198; Papahadjopoulos (1975) Biochim. Biophys. Acta 394:483; Wilson (1979) Cell 17:77); Deamer & Bangham (1976) Biochim. Biophys. Acta 443:629; Ostro (1977) Biochem. Biophys. Res. Commun. 76:836; Fraley (1979) Proc. Natl. Acad. Sci. USA 76:3348); Enoch & Strittmatter (1979) Proc. Natl. Acad. Sci. USA 76:145; Fraley (1980) J. Biol. Chem. (1980) 255:10431; Szoka & Papahadjopoulos (1978) Proc. Natl. Acad. Sci. USA 75:145; and Schaefer-Ridder (1982) Science 215:166.

E.Lipoproteins

In addition, lipoproteins can be included with the polynucleotide/polypeptide tobe delivered. Examples of lipoproteins to be utilized include: chylomicrons, HDL, IDL, LDL, and VLDL. Mutants, fragments, or fusions of these proteins can also be used. Also, modifications of naturally occurring lipoproteins can be used, such as acetylated LDL. These lipoproteins can target the delivery of polynucleotides to cells expressing lipoprotein receptors. Preferably, if lipoproteins are including with the polynucleotide to be delivered, no other targeting ligand is included in the composition.

Naturally occurring lipoproteins comprise a lipid and a protein portion. The protein portion are known as apoproteins. At the present, apoproteins A, B, C, D, and E have been isolated and identified. At least two of these contain several proteins, designated by Roman numerals, AI, AII, AIV; CI, CII, CIII.

A lipoprotein can comprise more than one apoprotein. For example, naturally occurring chylomicrons comprises of A, B, C & E, over time these lipoproteins lose A and acquire C & E. VLDL comprises A, B, C & E apoproteins, LDL comprises apoprotein B; and HDL comprises apoproteins A, C, & E.

The amino acid of these apoproteins are known and are described in, for example, Breslow (1985) Annu Rev. Biochem 54:699; Law (1986) Adv. Exp Med. Biol. 151:162; Chen (1986) JBiol Chem 261:12918; Kane (1980) Proc Natl Acad Sci USA 77:2465; and Utermann (1984) Hum Genet 65:232.

Lipoproteins contain a variety of lipids including, triglycerides, cholesterol (free and esters), and phospholipids. The composition of the lipids varies in naturally occurring lipoproteins. For example, chylomicrons comprise mainly triglycerides. A more detailed description of the lipid content of naturally occurring lipoproteins can be found, for example, in Meth. Enzymol. 128 (1986). The composition of the lipids are chosen to aid in conformation of the apoprotein for receptor binding activity. The composition of lipids can also be chosen to facilitate hydrophobic interaction and association with the polynucleotide binding molecule.

Naturally occurring lipoproteins can be isolated from serum by ultracentrifugation, for instance. Such methods are described in Meth. Enzymol. (supr*a)*; Pitas (1980) J. Biochem. 255:5454-5460 and Mahey (1979) J Clin. Invest 64: 743-750. Lipoproteins can also be produced by *in vitro* or recombinant methods by expression of the apoprotein genes in a desired host cell. See, for example, Atkinson (1986) Annu Rev Biophys Chem 15:403 and Radding (1958) Biochim Biophys Acta 30: 443. Lipoproteins can also be purchased from commercial suppliers, such as Biomedical Techniologies, Inc., Stoughton, MA, USA. Further description of lipoproteins can be found in WO98/06437..

F.Polycationic Agents

Polycationic agents can be included, with or without lipoprotein, in a composition with the desired polynucleotide/ polypeptide to be delivered.

Polycationic agents, typically, exhibit a net positive charge at physiological relevant pH and are capable of neutralizing the electrical charge of nucleic acids to facilitate delivery to a desired location. These agents have both in vitro, ex vivo, and in vivo applications. Polycationic agents can be used to deliver nucleic acids to a living subject either intramuscularly, subcutaneously, *etc.*

The following are examples of useful polypeptides as polycationic agents: polylysine, polyarginine, polyornithine, and protamine. Other examples include histones, protamines, human serum albumin, DNA binding proteins, non-histone chromosomal proteins, coat proteins from DNA viruses, such as (X174, transcriptional factors also contain domains that bind DNA and therefore may be useful as nucleic aid condensing agents. Briefly, transcriptional factors such as C/CEBP, c-jun, c-fos, AP-1, AP-2, AP-3, CPF, Prot-1, Sp-1, Oct-1, Oct-2, CREP, and TFIID contain basic domains that bind DNA sequences.

Organic polycationic agents include: spermine, spermidine, and purtrescine.

The dimensions and of the physical properties of a polycationic agent can be extrapolated from the list above, to construct other polypeptide polycationic agents or to produce synthetic polycationic agents.

Synthetic polycationic agents which are useful include, for example, DEAE-dextran, polybrene. Lipofectin™, and lipofectAMINE™ are monomers that form polycationic complexes when combined with polynucleotides/polypeptides.

*Immunodiagnostic Assays*

Streptococcus antigens of the invention can be used in immunoassays to detect antibody levels (or, conversely, anti-streptococcus antibodies can be used to detect antigen levels). Immunoassays based on well defined, recombinant antigens can be developed to replace invasive diagnostics methods. Antibodies to streptococcus proteins within biological samples, including for example, blood or serum samples, can be detected. Design of the immunoassays is subject to a great deal of variation, and a variety of these are known in the art. Protocols for the immunoassay may be based, for example, upon competition, or direct reaction, or sandwich type assays. Protocols may also, for example, use solid supports, or may be by immunoprecipitation. Most assays involve the use of labeled antibody or polypeptide; the labels may be, for example, fluorescent, chemiluminescent, radioactive, or dye molecules. Assays which amplify the signals from the probe are also known; examples of which are assays which utilize biotin and avidin, and enzyme-labeled and mediated immunoassays, such as ELISA assays.

Kits suitable for immunodiagnosis and containing the appropriate labeled reagents are constructed by packaging the appropriate materials, including the compositions of the invention, in suitable containers, along with the remaining reagents and materials (for example, suitable buffers, salt solutions, *etc.*) required for the conduct of the assay, as well as suitable set of assay instructions.

*Nucleic Acid Hybridisation*

"Hybridization" refers to the association of two nucleic acid sequences to one another by hydrogen bonding. Typically, one sequence will be fixed to a solid support and the other will be free in solution. Then, the two sequences will be placed in contact with one another under conditions that favor hydrogen bonding. Factors that affect this bonding include: the type and volume of solvent; reaction temperature; time of hybridization; agitation; agents to block the non-specific attachment of the liquid phase sequence to the solid support (Denhardt's reagent or BLOTTO); concentration of the sequences; use of compounds to increase the rate of association of sequences (dextran sulfate or polyethylene glycol); and the stringency of the washing conditions following hybridization. See Sambrook *et al.* [*supra*] Volume 2, chapter 9, pages 9.47 to 9.57.

"Stringency" refers to conditions in a hybridization reaction that favor association of very similar sequences over sequences that differ. For example, the combination of temperature and salt concentration should be chosen that is approximately 120 to 200°C below the calculated Tm of the hybrid under study. The temperature and salt conditions can often be determined empirically in preliminary experiments in whichsamples of genomic DNA immobilized on filters are hybridized to the sequence of interest and then washed under conditions of different stringencies. See Sambrook *et al.* at page 9.50.

Variables to consider when performing, for example, a Southern blot are(1) the complexity of the DNA being blotted and (2) the homology between the probe and the sequences being detected. The total amount of the fragment (s) to be studied can vary a magnitude of 10, from 0.1 to 1μg for a plasmid or phage digest to $10^{-9}$ to $10^{-8}$ g for a single copy gene in a highly complex eukaryotic genome. For lower complexity polynucleotides, substantially shorter blotting, hybridization, and exposure times, a smaller amount of starting polynucleotides, and lower specific activity of probes can be used. For example, a single-copy yeast gene can be detected with an exposure time of only 1 hour starting with 1 μg of yeast DNA, blotting for two hours, and hybridizing for 4-8 hours with a probe of $10^8$ cpm/μg. For a single-copy mammalian gene a conservative approach would start with 10 μg of DNA, blot overnight, and hybridize overnight in the presence of 10% dextran sulfate using a probe of greater than $10^8$ cpm/μg, resulting in an exposure time of ~24 hours.

Several factors can affect the melting temperature (Tm) of a DNA-DNA hybrid between the probe and the fragment of interest, and consequently, the appropriate conditions for hybridization and washing. In many cases the probe is not 100% homologous to the fragment. Other commonly encountered variables include the length and total G+C content of the hybridizing sequences and the ionic strength and formamide content of the hybridization buffer. The effects of all of these factors can be approximated by a single equation:

$$Tm = 81 + 16.6(\log_{10}Ci) + 0.4[\%(G + C)] - 0.6(\%formamide) - 600/n - 1.5(\%mismatch).$$

where Ci is the salt concentration (monovalent ions) and *n* is the length of the hybrid in base pairs (slightly modified from Meinkoth & Wahl (1984) Anal. Biochem. 138: 267-284).

In designing a hybridization experiment, some factors affecting nucleic acid hybridization can be conveniently altered. The temperature of the hybridization and washes and the salt concentration during the washes are the simplest to adjust. As the temperature of the hybridization increases (*ie*. stringency), it becomes less likely for hybridization to occur between strands that are nonhomologous, and as a result, background decreases. If the radiolabeled probe is not completely homologous with the immobilized fragment (as is frequently he case in gene family and interspecies hybridization experiments), the hybridization temperature must be reduced, and background will increase. The temperature of the washes affects the intensity of the hybridizing band and the degree of background in a similar manner. The stringency of the washes is also increased with decreasing salt concentrations.

In general, convenient hybridization temperatures in the presence of 50% formamide are 42°C for a probe with is 95% to 100% homologous to the target fragment, 37°C for 90% to 95% homology, and 32°C for 85% to 90% homology. For lower homologies, formamide content should be lowered and temperature adjusted accordingly, using the equation above. If the homology between the probe and the target fragment are not known, the simplest approach is to start with both hybridization and wash conditions which are nonstringent. If nonspecific bands or high background are observed after autoradiography, the filter can be washed at high stringency and reexposed. If the time required for exposure makes this approach impractical, several hybridization and/or washing stringencies should be tested in parallel.

*Nucleic Acid Probe Assays*

Methods such as PCR, branched DNA probe assays, or blotting techniques utilizing nucleic acid probes according to the invention can determine the presence of cDNA or mRNA. A probe is said to "hybridize" with a sequence of the invention if it can form a duplex or double stranded complex, which is stable enough to be detected.

The nucleic acid probes will hybridize to the streptococcus nucleotide sequences of the invention (including both sense and antisense strands). Though many different nucleotide sequences will encode the amino acid sequence, the native streptococcus sequence is preferred because it is the actual sequence present in cells. mRNA represents a coding sequence and so a probe should be complementary to the coding sequence; single-stranded cDNA is complementary to mRNA, and so a cDNA probe should be complementary to the non-coding sequence.

The probe sequence need not be identical to the streptococcus sequence (or its complement)- some variation in the sequence and length can lead to increased assay sensitivity if the nucleic acid probe can form a duplex with target nucleotides, which can be detected. Also, the nucleic acid probe can include additional nucleotides to stabilize the formed duplex. Additional streptococcus sequence may also be helpful as a label to detect the formed duplex. For example, a non-complementary nucleotide sequence may be attached to the 5' end of the probe, with the remainder of the probe sequence being complementary to a streptococcus sequence. Alternatively, non-complementary bases or longer sequences can be interspersed into the probe, provided that the probe sequence has sufficient complementarity with the a streptococcus sequence in order to hybridize therewith and thereby form a duplex which can be detected.

The exact length and sequence of the probe will depend on the hybridization conditions (*e.g.* temperature, salt condition *etc.*). For example, for diagnostic applications, depending on the complexity of the analyte sequence, the nucleic acid probe typically contains at least 10-20 nucleotides, preferably 15-25, and more preferably at least 30 nucleotides, although it may be shorter than this. Short primers generally require cooler temperatures to form sufficiently stable hybrid complexes with the template.

Probes may be produced by synthetic procedures, such as the triester method of Matteucci et al. [J. Am. Chem. Soc. (1981) 103:3185], or according to Urdea et al. [Proc. Natl. Acad. Sci. USA (1983) 80: 7461], or using commercially available automated oligonucleotide synthesizers.

The chemical nature of the probe can be selected according to preference. For certain applications, DNA or RNA are appropriate. For other applications, modifications may be incorporated *eg*. backbone modifications, such as phosphorothioates or methylphosphonates, can be used to increase *in vivo* half-life, alter RNA affinity, increase nuclease resistance *etc.* [*eg.* see Agrawal & Iyer (1995) Curr Opin Biotechnol 6:12-19; Agrawal (1996) TIBTECH 14:376-387]; analogues such as peptide nucleic acids may also be used [*eg.* see Corey (1997) TIBTECH 15:224-229; Buchardt et al. (1993) TIBTECH 11:384-386].

Alternatively, the polymerase chain reaction (PCR) is another well-known means for detecting small amounts of target nucleic acid. The assay is described in Mullis et al. [Meth. Enzymol. (1987) 155:335-350] & US patents 4,683,195 & 4,683,202. Two "primer" nucleotides hybridize with the target nucleic acids and are used to prime the reaction. The primers can comprise sequence that does not hybridize to the sequence of the amplification target (or its complement) to aid with duplex stability or, for example, to incorporate a convenient restriction site. Typically, such sequence will flank the desired streptococcus sequence.

A thermostable polymerase creates copies of target nucleic acids from the primers using the original target nucleic acids as a template. After a threshold amount of target nucleic acids are generated by the polymerase, they can be detected by more traditional methods, such as Southern blots. When using the Southern blot method, the labelled probe will hybridize to the streptococcus sequence (or its complement).

Also, mRNA or cDNA can be detected by traditional blotting techniques described in Sambrooket *al* [*supra*].

mRNA, or cDNA generated from mRNA using a polymerase enzyme, can be purified and separated using gel electrophoresis. The nucleic acids on the gel are then blotted onto a solid support, such as nitrocellulose. The solid support is exposed to a labelled probe and then washed to remove any unhybridized probe. Next, the duplexes containing the labeled probe are detected. Typically, the probe is labelled with a radioactive moiety.

## MODES FOR CARRYING OUT THE INVENTION

The genome sequence (2,162,598 base pairs) of a *S.pneumoniae* type 4 strain [Aaberge et al. (1995) Microbial Pathogenesis 18: 141-152] isolate previously referred to as: JNR.7/87 [Bricker et al. (1999) FEMS Microbiol. Lett. 172: 131]; KNR.7/87 [de Saizieu et al. (2000) J. Bacteriol. 182:4696; Hakenbeck et al. (2001) Infect. Immun. 69:2477]; and N4 [Wizemann et al. (2001) Infect. Immun. 69:1593] is set out in the sequence listing of WO-A-02/077021 as SEQ ID 4979 [see also Tettelin et al. (2001) Science 293:498].

2489 coding regions were identified within this sequence using GLIMMER2 [Delcher et al. (1999) Nucleic Acids Research 27:4636-4641]. The nucleic acid sequences are given in the sequence listing with odd numbers. For 2469 of the 2489 regions, amino acid sequences were inferred and, for these, the nucleic acid sequence is followed by its inferred translation product, together with an indication of cellular localisation, any sequence motifs of note, and an indication of similarity to any corresponding ORF in the Hoskins *et al.* R6 sequence. SEQ ID NO: 2 is: cell wall surface anchor family protein; Cellular location: Peptidoglycan-bound (LPXTG).

Various tests can be used to assess the *in vivo* immunogenicity of the proteins identified in the examples. For example, the proteins can be expressed recombinantly and used to screen patient sera by immunoblot. A positive reaction between the protein and patient serum indicates that the patient has previously mounted an immune response to the protein in question *i.e.* the protein is an immunogen. This method can also be used to identify immunodominant proteins.

The recombinant proteins can also be conveniently used to prepare antibodies *e.g.* in a mouse. These can be used for direct confirmation that a protein is located on the cell-surface. Labelled antibody (*e.g.* fluorescent labelling for FACS) can be incubated with intact bacteria and the presence of label on the bacterial surface confirms the location of the protein.

Of the 2489 coding regions, 1910 have homologs in *S.pneumoniae* strain R6 (Hoskins *et al.*). These 1910 regions can be used for multi-strain diagnosis and/or immunisation. Conversely, the remaining regions can be used to distinguish bacteria in strain R6.

Of the 2489 coding regions, 432 show homology to the 'GBS*nnn*' antigens listed in Table IV of WO-A-02/34771 and are thus inferred to be useful antigens for immunisation and/or diagnosis.

| SEQ ID | GBS*nnn* |
|---|---|
| 2 | GBS59 |

It will be understood that the invention has been described by way of example only and modifications may be made whilst remaining within the scope and spirit of the invention.

## Claims

**1.** A protein comprising:

(a) an amino acid sequence of SEQ ID NO: 2;
(b) an amino acid sequence having 50% or greater sequence identity to SEQ ID NO:2; or
(c) a fragment of an amino acid sequence of SEQ ID NO:2.

**2.** An antibody which binds to a protein according to claim 1.

**3.** A nucleic acid molecule which encodes a protein according to claim 1, or is complementary to a nucleic acid molecule which encodes a protein according to claim 1.

**4.** A nucleic acid molecule according to claim 3, comprising a nucleotide sequence of SEQ ID NO: 1.

**5.** A composition comprising a protein, a nucleic acid molecule, or an antibody according to any preceding claim, optionally wherein the composition comprises two or more proteins of claim 1.

**6.** A composition according to claim 5 being a vaccine composition or a diagnostic composition.

**7.** A composition according to claim 5 or claim 6 for use as a pharmaceutical.

**8.** A composition according to claim 7 for use in a method of treatment or prevention of a disease or infection due to streptococcus bacteria, particularly *S.pneumoniae.*

**9.** A composition according to claim 8, for the use of that claim, wherein the disease is meningitis, pneumonia, sepsis, otitis media or an ear infection.

**10.** A kit comprising primers for amplifying a target sequence contained within a Streptococcus nucleic acid sequence, the kit comprising a first primer and a second primer, wherein the first primer is substantially complementary to said target sequence and the second primer is substantially complementary to a complement of said target sequence, wherein the parts of said primers which have substantial complementarity define the termini of the target sequence to be amplified, and wherein the first and/or second primer is a nucleic acid according to claim 3 or claim 4.

**11.** A kit comprising first and second single-stranded oligonucleotides which allow amplification of a Streptococcus template nucleic acid sequence contained in a single- or double-stranded nucleic acid (or mixture thereof), wherein: (a) the first oligonucleotide comprises a primer sequence which is substantially complementary to said template nucleic acid sequence; (b) the second oligonucleotide comprises a primer sequence which is substantially complementary to the complement of said template nucleic acid sequence; (c) the first oligonucleotide and/or the second oligonucleotide comprise(s) sequence which is not complementary to said template nucleic acid; (d) said primer sequences define the termini of the template sequence to be amplified; and (e) the first and/or second oligonucleotide is a nucleic acid according claim 3 or claim 4.

**12.** A hybrid protein represented by the formula $NH_2A-[-X-L-]_n-B-COOH$, wherein X is the amino acid sequence of a protein according to claim 1, L is an optional linker amino acid sequence, A is an optional N-terminal amino acid sequence, B is an optional C-terminal amino acid sequence, and n is an integer greater than 1.

**13.** An assay comprising the steps of contacting a test compound with a protein according to claim 1, and determining whether the test compound binds to said protein.

**14.** The composition of claim 5 further comprising one or more of the following antigens:

- a protein antigen from *Helicobacter pylori* ;
- a protein antigen from *N.meningitidis* serogroup B;
- an outer-membrane vesicle (OMV) preparation from *N.meningitidis*;
- a saccharide antigen from *N.meningitidis* serogroup A, C, W 135 and/or Y;
- a saccharide antigen from *Streptococcus pneumoniae*;
- an antigen from hepatitis A virus;
- an antigen from hepatitis B virus;
- an antigen from hepatitis C virus;
- an antigen from *Bordetella pertussis*;
- a diphtheria antigen;
- a tetanus antigen;
- a saccharide antigen from *Haemophilus influenzae* B.
- an antigen from *N.gonorrhoeae;*
- an antigen from *Chlamydia pneumoniae;*
- an antigen from *Streptococcus agalactiae;*
- an antigen from *Streptococcus pyogenes* ;
- an antigen from *Chlamydia trachomatis* ;
- an antigen from *Porphyromonas gingivalis*;
- polio antigen(s);
- rabies antigen(s);
- measles, mumps and/or rubella antigens;
- influenza antigen(s);
- an antigen from Moraxella catarrhalis; and/or
- an antigen from Staphylococcus aureus.

Europäisches Patentamt
European Patent Office
Office européen des brevets

# EUROPEAN SEARCH REPORT

Application Number
EP 10 17 9935

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 00/06737 A2 (MICROBIAL TECHNICS LIMITED [GB]; GILBERT CHRISTOPHE FRANCOIS GU [GB];) 10 February 2000 (2000-02-10)<br>* SEQ NO. 4191.3 has 100% identity in a 1995nt overlap to SEQ ID NO.1 of the present application. SEQ NO. 127 has 100% identity in a 665aa overlap to SEQ ID NO.2 of the present application.;<br>page 65; claims 1,7,11,15,20; tables 1,2 *<br>* page 85 *<br>----- | 1-14 | INV.<br>C12N15/31<br>C12N1/21<br>C12Q1/68<br>A61K39/09<br>A61P31/04<br>C07K14/315<br>C07K16/12<br>G01N33/68 |
| X | WO 98/18931 A2 (HUMAN GENOME SCIENCES INC [US]; KUNSCH CHARLES A [US]; CHOI GIL H [US]) 7 May 1998 (1998-05-07)<br>* SEQ ID NO 54 has 100% identity in a 1995nt overlap to SEQ ID NO.1 of the present application.;<br>page 37 *<br>----- | 1-3,5-8 | |
| X,D,P | TETTELIN HERVE ET AL: "Complete genome sequence of a virulent isolate of Streptococcus pneumoniae.", SCIENCE (WASHINGTON D C), vol. 293, no. 5529, 2001, pages 498-506, XP002218261, ISSN: 0036-8075<br>* the whole document *<br>----- | 1-14 | TECHNICAL FIELDS SEARCHED (IPC)<br>C12N<br>C12Q<br>A61K<br>A61P<br>C07K<br>G01N |
| A | WIZEMANN T M ET AL: "Use of a whole genome approach to identify vaccine molecules affording protection against Streptococcus pneumoniae infection", INFECTION AND IMMUNITY, AMERICAN SOCIETY FOR MICROBIOLOGY, WASHINGTON, US LNKD- DOI:10.1128/IAI.69.3.1593-1598.2001, vol. 69, no. 3, 1 March 2001 (2001-03-01), pages 1593-1598, XP002263818, ISSN: 0019-9567<br>* the whole document *<br>----- | 1-14 | |

The present search report has been drawn up for all claims

1

| Place of search | Date of completion of the search | Examiner |
|---|---|---|
| Munich | 24 November 2010 | Weinberg, Suzanna |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 10 17 9935

This annex lists the patent family members relating to the patent documents cited in the above-mentioned European search report.
The members are as contained in the European Patent Office EDP file on
The European Patent Office is in no way liable for these particulars which are merely given for the purpose of information.

24-11-2010

| Patent document cited in search report | Publication date | Patent family member(s) | Publication date |
|---|---|---|---|
| WO 0006737 A2 | 10-02-2000 | AT 361365 T | 15-05-2007 |
| | | EP 2053126 A1 | 29-04-2009 |
| | | EP 1100921 A2 | 23-05-2001 |
| | | JP 2002531055 T | 24-09-2002 |
| | | JP 2008263966 A | 06-11-2008 |
| | | JP 2008263968 A | 06-11-2008 |
| | | JP 2008263969 A | 06-11-2008 |
| | | JP 2008263970 A | 06-11-2008 |
| | | JP 2009148283 A | 09-07-2009 |
| WO 9818931 A2 | 07-05-1998 | AT 479756 T | 15-09-2010 |
| | | AT 348887 T | 15-01-2007 |
| | | AU 773895 B2 | 10-06-2004 |
| | | AU 5194598 A | 22-05-1998 |
| | | AU 6909098 A | 22-05-1998 |
| | | CA 2269663 A1 | 07-05-1998 |
| | | CA 2271720 A1 | 07-05-1998 |
| | | DE 69737125 T2 | 25-10-2007 |
| | | DK 0942983 T3 | 30-04-2007 |
| | | EP 1400592 A1 | 24-03-2004 |
| | | EP 1770164 A2 | 04-04-2007 |
| | | EP 0942983 A2 | 22-09-1999 |
| | | EP 0941335 A2 | 15-09-1999 |
| | | ES 2277362 T3 | 01-07-2007 |
| | | JP 4469026 B2 | 26-05-2010 |
| | | JP 2001505415 T | 24-04-2001 |
| | | JP 2001501833 T | 13-02-2001 |
| | | JP 4467606 B2 | 26-05-2010 |
| | | JP 2008022854 A | 07-02-2008 |
| | | JP 2008035865 A | 21-02-2008 |
| | | JP 2008178407 A | 07-08-2008 |
| | | JP 2009039110 A | 26-02-2009 |
| | | PT 942983 E | 28-02-2007 |
| | | WO 9818930 A2 | 07-05-1998 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0509612 B
- EP 0505012 B
- GB 0104789 W
- WO 9804702 A
- WO 9924578 A
- WO 9936544 A
- WO 9957280 A
- WO 0022430 A
- WO 9629412 A
- WO 0152885 A
- WO 0202606 A
- WO 9927105 A
- WO 0027994 A
- WO 0037494 A
- WO 9928475 A
- EP 0372501 A
- EP 0378881 A
- EP 0427347 A
- WO 9317712 A
- WO 9858668 A
- EP 0471177 A
- WO 0056360 A
- WO 0061761 A
- US 5753235 A
- EP 127839 A
- EP 155476 A
- WO 89046699 A
- US 5693506 A
- US 5659122 A
- US 5608143 A
- US 4738921 A
- EP 0036776 A
- EP 0121775 A
- US 4689406 A
- US 4551433 A
- EP 0267851 A
- EP 0219237 A
- EP 0324647 A
- US 4336336 A
- EP 0244042 A
- EP 0127328 A
- EP 0036259 A
- EP 0063953 A
- WO 8404541 A
- EP 0136829 A
- EP 0136907 A
- US 4745056 A
- EP 0284044 A
- EP 0329203 A
- US 4876197 A
- US 4880734 A
- EP 0164556 A
- EP 0196056 A
- WO 88024066 A
- EP 0012873 A
- JP O62096086 B
- US 4588684 A
- EP 0060057 A
- US 4546083 A
- US 4870008 A
- EP 0324274 A
- WO 8902463 A
- US 4837148 A
- US 4929555 A
- WO 9820734 A
- WO 9014837 A
- US 5591624 A
- WO 9637626 A
- WO 9530763 A
- WO 9205266 A
- GB 2200651 A
- EP 0415731 A
- EP 0345242 A
- EP 0334301 A
- WO 8902468 A
- WO 8905349 A
- WO 8909271 A
- WO 9002806 A
- WO 9007936 A
- WO 9403622 A
- WO 9325698 A
- WO 9325234 A
- WO 9311230 A
- WO 9310218 A
- WO 9102805 A
- WO 9102825 A
- WO 9507994 A
- US 5219740 A
- US 4405712 A
- US 4861719 A
- US 4980289 A
- US 4777127 A
- WO 9307283 A
- WO 9306223 A
- WO 9307282 A
- WO 9412649 A
- WO 9303769 A
- WO 9319191 A

- WO 9428938 A
- WO 9511984 A
- WO 9500655 A
- WO 9527071 A
- WO 9529993 A
- WO 9534671 A
- WO 9605320 A
- WO 9408026 A
- WO 9411506 A
- WO 9424299 A
- WO 9514102 A
- WO 9524297 A
- WO 9502697 A
- WO 9428152 A
- WO 9509241 A
- WO 9525807 A
- WO 9505835 A
- WO 9418922 A
- WO 9509654 A
- WO 9309239 A
- US 5478745 A
- US 4797368 A, Carter
- US 5139941 A, Muzyczka
- US 5474935 A, Chartejee
- WO 94288157 A, Kotin
- US 5354678 A
- US 5173414 A
- US 5252479 A
- US 5288641 A
- EP 0176170 A, Roizman
- WO 9504139 A
- WO 9009441 A
- WO 9207945 A
- EP 0453242 A
- US 5091309 A
- US 5217879 A
- WO 9210578 A
- US 08405627 B
- WO 9421792 A
- US SN08679640 A
- US 4603112 A
- US 4769330 A
- WO 8901973 A
- US 5166057 A
- EP 0386882 A
- EP 0440219 A
- US 08366787 B
- US 08240030 B
- US 08404796 B
- US 5149655 A
- US 5206152 A
- WO 9211033 A
- US 60023867 B
- WO 9011092 A
- US 5580859 A
- US 5422120 A
- WO 9513796 A
- WO 9423697 A
- WO 9114445 A
- EP 524968 A
- US SN60023867 P
- US 4762915 A
- EP 0524968 A
- WO 9314778 A
- WO 9806437 A
- US 4683195 A
- US 4683202 A
- WO 02077021 A
- WO 0234771 A

**Non-patent literature cited in the description**


- **Breedveld.** *Lancet,* 2000, vol. 355 (9205), 735-740
- **Gorman ; Clark.** *Semin. Immunol.,* 1990, vol. 2, 457-466
- **Glezen ; Alpers.** *Clin. Infect. Dis.,* 1999, vol. 28, 219-224
- **Hoskins et al.** *J Bacteriol,* 2001, vol. 183, 5709-17
- **Cherian et al.** *J. Clin. Microbiol.,* 1998, vol. 36, 3605-3608
- **Kearns et al.** *J. Clin. Microbiol.,* 1999, vol. 37, 3434
- **Borodovsky ; McIninch.** *Computers Chem.,* 1993, vol. 17, 122-133
- **Salzberg et al.** *Nucleic Acids Res.,* 1998, vol. 26, 544-548
- **Salzberg et al.** *Genomics,* 1999, vol. 59, 24-31
- **Delcher et al.** *Nucleic Acids Res.,* 1999, vol. 27, 4636-4641
- **Shmatkov et al.** *Bioinformatics,* 1999, vol. 15, 874-876
- **Tettelin et al.** *Science,* 2000, vol. 287, 1809-1815
- **Pizza et al.** *Science,* 2000, vol. 287, 1816-1820
- **Bjune et al.** *Lancet,* 1991, vol. 338 (8775), 1093-1096
- **Fukasawa et al.** *Vaccine,* 1999, vol. 17, 2951-2958
- **Rosenqvist et al.** *Dev. Biol. Stand.,* 1998, vol. 92, 323-333
- **Costantino et al.** *Vaccine,* 1992, vol. 10, 691-698
- **Costantino et al.** *Vaccine,* 1999, vol. 17, 1251-1263

- **Watson.** *Pediatr Infect Dis J,* 2000, vol. 19, 331-332
- **Rubin.** *Pediatr Clin North Am,* 2000, vol. 47, 269-285
- **Jedrzejas.** *Microbiol Mol Biol Rev,* 2001, vol. 65, 187-207
- **Bell.** *Pediatr Infect Dis J,* 2000, vol. 19, 1187-1188
- **Iwarson.** *APMIS,* 1995, vol. 103, 321-326
- **Gerlich et al.** *Vaccine,* 1990, vol. 8, 63-6879-80
- **Hsu et al.** *Clin Liver Dis,* 1999, vol. 3, 901-915
- **Gustafsson et al.** *N. Engl. J. Med.,* 1996, vol. 334, 349-355
- **Rappuoli et al.** *TIBTECH,* 1991, vol. 9, 232-238
- Vaccines
- **Del Guidice et al.** *Molecular Aspects of Medicine,* 1998, vol. 19, 1-70
- **Kalman et al.** *Nature Genetics,* 1999, vol. 21, 385-389
- **Read et al.** *Nucleic Acids Res,* 2000, vol. 28, 1397-406
- **Shirai et al.** *J. Infect. Dis.,* 2000, vol. 181 (3), S524-S527
- **Ross et al.** *Vaccine,* 2001, vol. 19, 4135-4142
- **Sutter et al.** *Pediatr Clin North Am,* 2000, vol. 47, 287-308
- **Zimmerman ; Spann.** *Am Fam Physician,* 1999, vol. 59, 113-118125-126
- **Dreesen.** *Vaccine,* 1997, vol. 15, 2-6
- *MMWR Morb Mortal Wkly Rep,* 16 January 1998, vol. 47 (1), 12, 19
- **McMichael.** *Vaccine,* 2000, vol. 19 (1), 101-107
- **Kuroda et al.** *Lancet,* 2001, vol. 357 (9264), 1225-1240
- **Ramsay et al.** *Lancet,* 2001, vol. 357 (9251), 195-196
- **Lindberg.** *Vaccine,* 1999, vol. 17 (2), 28-36
- Conjugate Vaccines. vol. 10, 48-114
- **Sambrook.** Molecular Cloning; A Laboratory Manual. 1989, vol. I,II
- DNA Cloning. 1985, vol. I, II
- Oligonucleotide Synthesis. 1984
- Nucleic Acid Hybridization. 1984
- Transcription and Translation. 1984
- Animal Cell Culture. 1986
- Immobilized Cells and Enzymes. IRL Press, 1986
- **B. Perbal.** A Practical Guide to Molecular Cloning. 1984
- the Methods in Enzymology series. Academic Press, Inc, vol. 154, 155
- Gene Transfer Vectors for Mammalian Cells. Cold Spring Harbor Laboratory, 1987
- Immunochemical Methods in Cell and Molecular Biology. Academic Press, 1987
- **Scopes.** Protein Purification: Principles and Practice. Springer-Verlag, 1987
- Handbook of Experimental Immunology. 1986, vol. I-IV
- Expression of Cloned Genes in Mammalian Cells. **Sambrook et al.** Molecular Cloning: A Laboratory Manual. 1989
- **Maniatis et al.** *Science,* 1987, vol. 236, 1237
- **Alberts et al.** Molecular Biology of the Cell. 1989
- **Dijkema et al.** *EMBO J.,* 1985, vol. 4, 761
- **Gorman et al.** *Proc. Natl. Acad. Sci.,* 1982, vol. 79, 6777
- **Boshart et al.** *Cell,* 1985, vol. 41, 521
- **Sassone-Corsi ; Borelli.** *Trends Genet.,* 1986, vol. 2, 215
- **Birnstiel et al.** *Cell,* 1985, vol. 41, 349
- Termination and 3' end processing of eukaryotic RNA. **Proudfoot ; Whitelaw.** Transcription and splicing. 1988
- **Proudfoot.** *Trends Biochem. Sci.,* 1989, vol. 14, 105
- Expression of cloned genes in cultured mammalian cells. **Sambrook et al.** Molecular Cloning: A Laboratory Manual. 1989
- **Gluzman.** *Cell,* 1981, vol. 23, 175
- **Kaufman et al.** *Mol. Cell. Biol.,* 1989, vol. 9, 946
- **Shimizu et al.** *Mol. Cell. Biol.,* 1986, vol. 6, 1074
- **Summers ; Smith.** *Texas Agricultural Experiment Station Bulletin,* 1987
- **Miller et al.** *Ann. Rev. Microbiol.,* 1988, vol. 42, 177
- The Regulation of Baculovirus Gene Expression. **Friesen et al.** The Molecular Biology of Baculoviruses. 1986
- **Vlak et al.** *J. Gen. Virol.,* 1988, vol. 69, 765
- **Carbonell et al.** *Gene,* 1988, vol. 73, 409
- **Maeda et al.** *Nature,* 1985, vol. 315, 592
- **Lebacq-Verheyden et al.** *Molec. Cell. Biol.,* 1988, vol. 8, 3129
- **Smith et al.** *Proc. Nat'l Acad. Sci. USA,* 1985, vol. 82, 8404
- **Miyajima et al.** *Gene,* 1987, vol. 58, 273
- **Martin et al.** *DNA,* 1988, vol. 7, 99
- **Smith et al.** *Mol. Cell. Biol.,* 1983, vol. 3, 2156
- **Miller et al.** *Bioessays,* 1989, vol. 4, 91
- Current Protocols in Microbiology. 1990, vol. 2, 16.8
- **Carbonell et al.** *J. Virol.,* 1985, vol. 56, 153
- **Wright.** *Nature,* 1986, vol. 321, 718
- **Fraser et al.** *In Vitro Cell. Dev. Biol.,* 1989, vol. 25, 225
- **Zenk.** *Phytochemistry,* 1991, vol. 30, 3861-3863

- **Vaulcombe et al.** *Mol. Gen. Genet.,* 1987, vol. 209, 33-40
- **Chandler et al.** *Plant Molecular Biology,* 1984, vol. 3, 407-418
- **Rogers.** *J. Biol. Chem.,* 1985, vol. 260, 3731-3738
- **Rothstein et al.** *Gene,* 1987, vol. 55, 353-356
- **Whittier et al.** *Nucleic Acids Research,* 1987, vol. 15, 25152535
- **Wirsel et al.** *Molecular Microbiology,* 1989, vol. 3, 3-14
- **Yu et al.** *Gene,* 1992, vol. 122, 247-253
- Gibberellins. **R.L. Jones ; J. MacMillin.** Advanced Plant Physiology. Pitman Publishing Limited, 1984, 21-52
- **Sheen.** *Plant Cell,* 1990, vol. 2, 1027-1038
- **Maas et al.** *EMBO J.,* 1990, vol. 9, 3447-3452
- **Benkel ; Hickey.** *Proc. Natl. Acad. Sci.,* 1987, vol. 84, 1337-1339
- **Wilmink ; Dons.** *Plant Mol. Biol. Reptr,* 1993, vol. 11 (2), 165-185
- **Reed ; Maniatis.** *Cell,* 1985, vol. 41, 95-105
- **Crossway.** *Mol. Gen. Genet,* 1985, vol. 202, 179-185
- **Krens et al.** *Nature,* 1982, vol. 296, 72-74
- **Klein et al.** *Nature,* 1987, vol. 327, 70-73
- **Knudsen ; Muller.** *Planta,* 1991, vol. 185, 330-336
- **Fraley et al.** *Proc. Natl. Acad. Sci. USA,* 1982, vol. 79, 1859-1863
- **Fromm et al.** *Proc. Natl Acad. Sci. USA,* 1985, vol. 82, 5824
- **Raibaud et al.** *Annu. Rev. Genet.,* 1984, vol. 18, 173
- **Chang et al.** *Nature,* 1977, vol. 198, 1056
- **Goeddel et al.** *Nuc. Acids Res.,* 1980, vol. 8, 4057
- **Yelverton et al.** *Nucl. Acids Res.,* 1981, vol. 9, 731
- The cloning of interferon and other mistakes. **Weissmann.** Interferon. 1981, vol. 3
- **Shimatake et al.** *Nature,* 1981, vol. 292, 128
- **Amann et al.** *Gene,* 1983, vol. 25, 167
- **de Boer et al.** *Proc. Natl. Acad. Sci.,* 1983, vol. 80, 21
- **Studier et al.** *J. Mol. Biol.,* 1986, vol. 189, 113
- **Tabor et al.** *Proc Natl. Acad. Sci.,* 1985, vol. 82, 1074
- **Shine et al.** *Nature,* 1975, vol. 254, 34
- Genetic signals and nucleotide sequences in messenger RNA. **Steitz et al.** Biological Regulation and Development: Gene Expression. 1979
- Expression of cloned genes in Escherichia coli. **Sambrook et al.** Molecular Cloning: A Laboratory Manual. 1989
- **Nagai et al.** *Nature,* 1984, vol. 309, 810
- **Jia et al.** *Gene,* 1987, vol. 60, 197
- **Allen et al.** *J. Biotechnol.,* 1987, vol. 5, 93
- **Makoff et al.** *J. Gen. Microbiol.,* 1989, vol. 135, 11
- **Miller et al.** *Bio/Technology,* 1989, vol. 7, 698
- **Masui et al.** *Experimental Manipulation of Gene Expression,* 1983
- **Ghrayeb et al.** *EMBO J.,* 1984, vol. 3, 2437
- **Oka et al.** *Proc. Natl. Acad. Sci.,* 1985, vol. 82, 7212
- **Palva et al.** *Proc. Natl. Acad. Sci. USA,* 1982, vol. 79, 5582
- **Davies et al.** *Annu. Rev. Microbiol.,* 1978, vol. 32, 469
- **Amann et al.** *Gene,* 1985, vol. 40, 183
- **Powell et al.** *Appl. Environ. Microbiol.,* 1988, vol. 54, 655
- **Masson et al.** *FEMS Microbiol. Lett.,* 1989, vol. 60, 273
- **Miller et al.** *Proc. Natl. Acad. Sci.,* 1988, vol. 85, 856
- **Wang et al.** *J. Bacteriol.,* 1990, vol. 172, 949
- **Cohen et al.** *Proc. Natl. Acad. Sci.,* 1973, vol. 69, 2110
- **Dower et al.** *Nucleic Acids Res.,* 1988, vol. 16, 6127
- An improved method for transformation of Escherichia coli with ColEI-derived plasmids. **Kushner.** Genetic Engineering: Proceedings of the International Symposium on Genetic Engineering. 1978
- **Mandel et al.** *J. Mol. Biol.,* 1970, vol. 53, 159
- **Taketo.** *Biochim. Biophys. Acta,* 1988, vol. 949, 318
- **Chassy et al.** *FEMS Microbiol. Lett.,* 1987, vol. 44, 173
- **Fiedler et al.** *Anal. Biochem,* 1988, vol. 170, 38
- **Augustin et al.** *FEMS Microbiol. Lett.,* 1990, vol. 66, 203
- **Barany et al.** *J. Bacteriol.,* 1980, vol. 144, 698
- Transformation of Streptococcus lactis by electroporation. **Harlander.** Streptococcal Genetics. 1987
- **Perry et al.** *Infect. Immun.,* 1981, vol. 32, 1295
- **Somkuti et al.** *Proc. 4th Evr. Cong. Biotechnology,* 1987, vol. 1, 412
- **Myanohara et al.** *Proc. Natl. Acad. Sci. USA,* 1983, vol. 80, 1
- **Cohen et al.** *Proc. Natl. Acad. Sci. USA,* 1980, vol. 77, 1078
- **Henikoff et al.** *Nature,* 1981, vol. 283, 835
- **Hollenberg et al.** *Curr. Topics Microbiol. Immunol.,* 1981, vol. 96, 119
- The Expression of Bacterial Antibiotic Resistance Genes in the Yeast Saccharomyces cerevisiae. **Hollenberg et al.** Plasmids of Medical, Environmental and Commercial Importance. 1979

- **Mercerau-Puigalon et al.** *Gene,* 1980, vol. 11, 163
- **Panthier et al.** *Curr. Genet.,* 1980, vol. 2, 109
- **Botstein et al.** *Gene,* 1979, vol. 8, 17-24
- **Brake et al.** *Proc. Natl. Acad. Sci USA,* 1984, vol. 81, 4642-4646
- **Stinchcomb et al.** *J. Mol. Biol.,* 1982, vol. 158, 157
- **Orr-Weaver et al.** *Methods in Enzymol.,* 1983, vol. 101, 228-245
- **Rine et al.** *Proc. Natl. Acad. Sci. USA,* 1983, vol. 80, 6750
- **Butt et al.** *Microbiol, Rev.,* 1987, vol. 51, 351
- **Kurtz et al.** *Mol. Cell. Biol.,* 1986, vol. 6, 142
- **Kunze et al.** *J. Basic Microbiol.,* 1985, vol. 25, 141
- **Gleeson et al.** *J. Gen. Microbiol.,* 1986, vol. 132, 3459
- **Roggenkamp et al.** *Mol. Gen. Genet.,* 1986, vol. 202, 302
- **Das et al.** *J. Bacteriol.,* 1984, vol. 158, 1165
- **De Louvencourt et al.** *J. Bacteriol.,* 1983, vol. 154, 737
- **Van den Berg et al.** *Bio/Technology,* 1990, vol. 8, 135
- **Cregg et al.** *Mol. Cell. Biol.,* 1985, vol. 5, 3376
- **Hinnen et al.** *Proc. Natl. Acad. Sci. USA,* 1978, vol. 75, 1929
- **Ito et al.** *J. Bacteriol.,* 1983, vol. 153, 163
- **Beach ; Nurse.** *Nature,* 1981, vol. 300, 706
- **Davidow et al.** *Curr. Genet,* 1985, vol. 10, 380471
- **Gaillardin et al.** *Curr. Genet,* 1985, vol. 10, 49
- **De Louvencourt et al.** *J. Bacteriol.,* 1983, vol. 154, 1165
- **Hinnenet.** *Proc. Natl. Acad. Sci. USA,* 1978, vol. 75, 1929
- **Davidow et al.** *Curr. Genet.,* 1985, vol. 10, 39
- **Gaillardin et al.** *Curr. Genet.,* 1985, vol. 10, 49
- **Kohler ; Milstein.** *Nature,* 1975, vol. 256, 495-96
- Remington's Pharmaceutical Sciences. Mack Pub. Co, 1991
- Vaccine design: the subunit and adjuvant approach. Plenum Press, 1995
- **Robinson ; Torres.** *Seminars in Immunol,* 1997, vol. 9, 271-283
- **Donnelly et al.** *Annu Rev Immunol,* 1997, vol. 15, 617-648
- **Jolly.** *Cancer Gene Therapy,* 1994, vol. 1, 51-64
- **Kimura.** *Human Gene Therapy,* 1994, vol. 5, 845-852
- **Connelly.** *Human Gene Therapy,* 1995, vol. 6, 185-193
- **Kaplitt.** *Nature Genetics,* 1994, vol. 6, 148-153
- **O'Neill.** *J. Virol.,* 1985, vol. 53, 160
- **Kelly.** *J. Virol.,* 1983, vol. 45, 291
- RNA Tumor Viruses. Cold Spring Harbor Laboratory, 1985
- **Hartley ; Rowe.** *J Virol,* 1976, vol. 19, 19-25
- **Vile.** *Cancer Res,* 1993, vol. 53, 3860-3864
- **Vile.** *Cancer Res,* 1993, vol. 53, 962-967
- **Ram.** *Cancer Res,* 1993, vol. 53, 83-88
- **Takamiya.** *J Neurosci Res,* 1992, vol. 33, 493-503
- **Baba.** *J Neurosurg,* 1993, vol. 79, 729-735
- **Mann.** *Cell,* 1983, vol. 33, 153
- **Cane.** *Proc Natl Acad Sci,* 1984, vol. 81, 6349
- **Miller.** *Human Gene Therapy,* 1990, 1
- **Berkner.** *Biotechniques,* 1988, vol. 6, 616
- **Rosenfeld.** *Science,* 1991, vol. 252, 431
- **Curiel.** *Hum. Gene Ther.,* 1992, vol. 3, 147-154
- **Nahreini.** *Gene,* 1993, vol. 124, 257-262
- **Samulski.** *J. Virol.,* 1987, vol. 61, 3096
- **Su.** *Human Gene Therapy,* 1996, vol. 7, 463-470
- **Geller.** *Science,* 1988, vol. 241, 1667-1669
- **Fink.** *Human Gene Therapy,* 1992, vol. 3, 11-19
- **Evans.** *Nature,* 1989, vol. 339, 385
- **Arnold.** *J Cell Biochem,* 1990, L401
- **Fisher-Hoch.** *Proc Natl Acad Sci,* 1989, vol. 86, 317
- **Flexner.** *Ann NY Acad Sci,* 1989, vol. 569, 86
- **Flexner.** *Vaccine,* 1990, vol. 8, 17
- **Mulligan.** *Nature,* 1979, vol. 277, 108
- **Madzak.** *J Gen Virol,* 1992, vol. 73, 1533
- **Enami.** *Proc Natl Acad Sci,* 1990, vol. 87, 3802-3805
- **Enami ; Palese.** *J Virol,* 1991, vol. 65, 2711-2713
- **Luytjes.** *Cell,* 1989, vol. 59, 110
- **McMichael.** *NEJ Med,* 1983, vol. 309, 13
- **Yap.** *Nature,* 1978, vol. 273, 238
- *Nature,* 1979, vol. 277, 108
- **Buchschacher.** *J. Virol.,* 1992, vol. 66, 2731
- **Hamre.** *Proc Soc Exp Biol Med,* 1966, vol. 121, 190
- **Curiel.** *Hum Gene Ther,* 1992, vol. 3, 147-154
- **Wu.** *J Biol Chem,* 1989, vol. 264, 16985-16987
- **Philip.** *Mol Cell Biol,* 1994, vol. 14, 2411-2418
- **Woffendin.** *Proc Natl Acad Sci,* 1994, vol. 91, 1581-1585
- **Wu ; Wu.** *J. Biol. Chem.,* 1987, vol. 262, 4429-4432

- **Hucked.** *Biochem Pharmacol,* 1990, vol. 40, 253-263
- **Plank.** *Bioconjugate Chem,* 1992, vol. 3, 533-539
- **Woffendin et al.** *Proc. Natl. Acad. Sci. USA,* 1994, vol. 91 (24), 11581-11585
- **Stryer.** Biochemistry. W.H. Freeman, 1975, 236-240
- **Szoka.** *Biochem Biophys Acta,* 1980, vol. 600, 1
- **Bayer.** *Biochem Biophys Acta,* 1979, vol. 550, 464
- **Rivnay.** *Meth Enzymol,* 1987, vol. 149, 119
- **Wang.** *Proc Natl Acad Sci,* 1987, vol. 84, 7851
- **Plant.** *Anal Biochem,* vol. 176, 420
- **Hug ; Sleight.** *Biochim. Biophys. Acta,* 1991, vol. 1097, 1-17
- **Straubinger.** *Meth. Enzymol.,* 1983, vol. 101, 512-527
- **Felgner.** *Proc. Natl. Acad. Sci. USA,* 1987, vol. 84, 7413-7416
- **Malone.** *Proc. Natl. Acad. Sci. USA,* 1989, vol. 86, 6077-6081
- **Debs.** *J. Biol. Chem.,* 1990, vol. 265, 10189-10192
- **Szoka.** *Proc. Natl. Acad. Sci. USA,* 1978, vol. 75, 4194-4198
- **Straubinger.** *Meth. Immunol.,* 1983, vol. 101, 512-527
- **Papahadjopoulos.** *Biochim. Biophys. Acta,* 1975, vol. 394, 483
- **Wilson.** *Cell,* 1979, vol. 17, 77
- **Deamer ; Bangham.** *Biochim. Biophys. Acta,* 1976, vol. 443, 629
- **Ostro.** *Biochem. Biophys. Res. Commun.,* 1977, vol. 76, 836
- **Fraley.** *Proc. Natl. Acad. Sci. USA,* 1979, vol. 76, 3348
- **Enoch ; Strittmatter.** *Proc. Natl. Acad. Sci. USA,* 1979, vol. 76, 145
- **Fraley.** *J. Biol. Chem.,* 1980, vol. 255, 10431
- **Szoka ; Papahadjopoulos.** *Proc. Natl. Acad. Sci. USA,* 1978, vol. 75, 145
- **Schaefer-Ridder.** *Science,* 1982, vol. 215, 166
- **Breslow.** *Annu Rev. Biochem,* 1985, vol. 54, 699
- **Law.** *Adv. Exp Med. Biol.,* 1986, vol. 151, 162
- **Chen.** *JBiol Chem,* 1986, vol. 261, 12918
- **Kane.** *Proc Natl Acad Sci USA,* 1980, vol. 77, 2465
- **Utermann.** *Hum Genet,* 1984, vol. 65, 232
- *Meth. Enzymol.,* 1986, 128
- *Meth. Enzymol.*
- **Pitas.** *J. Biochem.,* 1980, vol. 255, 5454-5460
- **Mahey.** *J Clin. Invest,* 1979, vol. 64, 743-750
- **Atkinson.** *Annu Rev Biophys Chem,* 1986, vol. 15, 403
- **Radding.** *Biochim Biophys Acta,* 1958, vol. 30, 443
- **Meinkoth ; Wahl.** *Anal. Biochem.,* 1984, vol. 138, 267-284
- **Matteucci et al.** *J. Am. Chem. Soc.,* 1981, vol. 103, 3185
- **Urdea et al.** *Proc. Natl. Acad. Sci. USA,* 1983, vol. 80, 7461
- **Agrawal ; Iyer.** *Curr Opin Biotechnol,* 1995, vol. 6, 12-19
- **Agrawal.** *TIBTECH,* 1996, vol. 14, 376-387
- **Corey.** *TIBTECH,* 1997, vol. 15, 224-229
- **Buchardt et al.** *TIBTECH,* 1993, vol. 11, 384-386
- **Mullis et al.** *Meth. Enzymol.,* 1987, vol. 155, 335-350
- **Aaberge et al.** *Microbial Pathogenesis,* 1995, vol. 18, 141-152
- **Bricker et al.** *FEMS Microbiol. Lett.,* 1999, vol. 172, 131
- **de Saizieu et al.** *J. Bacteriol.,* 2000, vol. 182, 4696
- **Hakenbeck et al.** *Infect. Immun.,* 2001, vol. 69, 2477
- **Wizemann et al.** *Infect. Immun.,* 2001, vol. 69, 1593
- **Tettelin et al.** *Science,* 2001, vol. 293, 498
- **Delcher et al.** *Nucleic Acids Research,* 1999, vol. 27, 4636-4641